(19) Europäisches Patentamt
European Patent Office
Office européen des brevets

(11) Veröffentlichungsnummer: **0 125 636**
**B1**

(12) # EUROPÄISCHE PATENTSCHRIFT

(45) Veröffentlichungstag der Patentschrift: **01.04.87**

(51) Int. Cl.⁴: **C 07 D 237/14**, C 07 D 237/18, A 61 K 31/50

(21) Anmeldenummer: **84105291.3**

(22) Anmeldetag: **10.05.84**

(54) **Pyridazinone, ihre Herstellung und Verwendung, Pyridazinone enthaltende Arzneimittel.**

(30) Priorität: **11.05.83 CH 2581/83**

(43) Veröffentlichungstag der Anmeldung: **21.11.84 Patentblatt 84/47**

(45) Bekanntmachung des Hinweises auf die Patenterteilung: **01.04.87 Patentblatt 87/14**

(84) Benannte Vertragsstaaten: **AT BE CH DE FR GB IT LI LU NL SE**

(56) Entgegenhaltungen:
**FR - A - 2 383 175**
**US - A - 4 397 854**

**CHEMICAL ABSTRACTS, Band 98, 1983, Seite 64, Nr. 137630y, Columbus; ohio, US**

(73) Patentinhaber: **Byk Gulden Lomberg Chemische Fabrik GmbH, Byk-Gulden-Strasse 2, D-7750 Konstanz (DE)**

(72) Erfinder: **Amschler, Hermann, Dr., Hohenhewenstrasse 19, D-7760 Radolfzell (DE)**
Erfinder: **Ulrich, Wolf-Rüdiger, Dr., Brandesstrasse 14, D-7750 Konstanz (DE)**
Erfinder: **Eltze, Manfrid, Dr., Schützenstrasse 20, D-7750 Konstanz (DE)**
Erfinder: **Kilian, Ulrich, Dr., Stettinerstrasse 22, D-7750 Konstanz (DE)**
Erfinder: **Sanders, Karl, Dr., Felchengang 23, D-7750 Konstanz (DE)**
Erfinder: **Kolassa, Norbert, Prof. Dr., Lerchenweg 12, D-7750 Konstanz 19 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Technisches Gebiet

Die Erfindung betrifft Pyridazinone, ihre Herstellung und Verwendung und Pyridazinone enthaltende Arzneimittel.

Stand der Technik

6-Aryl-3[2H]pyridazinone als Ausgangsmaterialien oder Zwischenprodukte für die Synthese von Pharmazeutika und Pflanzenschutzmitteln sowie Verfahren zu ihrer Herstellung werden beispielsweise beschrieben von Baddar et al. [J. Chem. Soc. 1965, 3342), Steck (J. Heterocycl. Chem. 11(1974)755, Albright et al. [J.Heterocycl. Chem. 15(1978)881], Schreiber et al. [Bull. Soc. Chim. France 2(1973)625], Pitarch et al. [Eur. J. Med. Chem.- Chimica Therapeutica 9(1974)644] und Curran et al. [J. Med. Chem. 17(1974)273] oder sind u.a. aus folgenden Beschreibungen bekannt DE-OS 24 35 244, DE-OS 24 45 681, DE-OS 27 57 923.

6-Aryl-3[2H]pyridazinone mit bestimmter Wirkung sind z.B. aus folgenden Beschreibungen bekannt DE-OS 24 27 943, DE-OS 28 10 267, DE-OS 28 45 220, EP-OS 8391, EP-OS 10 156, JA-OS 58 008 015 und US-PS 4 397 854.

6-(3,4-Dimethoxyphenyl)-3[2H]pyridazinon wird von Pitarch et al. und in der DE-OS 28 10 267 beschrieben.

Darstellung der Erfindung

Bestimmte 6-Aryl-3[2H]pyridazinone der allgemeinen Formel I weisen nun eine vorteilhafte pharmakologische Wirkung auf.

Gegenstand der Erfindung sind 6-Aryl-3[2H]pyridazinone der allgemeinen Formel I

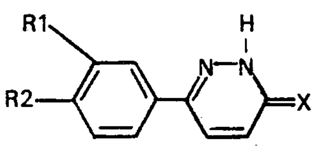

(I),

worin einer der Substituenten R1 oder R2 eine Methoxygruppe und der andere eine Alkoxygruppe mit 2 bis 5 Kohlenstoffatomen oder eine Alkenyloxygruppe mit 3 bis 5 Kohlenstoffatomen und X ein Sauerstoffatom oder ein Schwefelatom bedeuten, und ihre pharmakologisch verträglichen Salze mit Basen.

Alkoxy und Alkenyloxy sind geradkettig oder verzweigt. Die Doppelbindung von Alkenyloxy geht nicht von dem Kohlenstoffatom aus, das an das Sauerstoffatom bindet. Als Beispiele für Alkoxy und Alkenyloxy seien genannt n-Butoxy-, n-Propoxy, Ethoxy, Amyloxy, 2,2-Dimethylpropyloxy, Isopentyloxy, Isobutoxy, sek.-Butoxy, Isopropoxy, Buten-2-yloxy, Allyloxy, Methallyloxy; bevorzugte Alkoxy enthalten 3 oder 4 Kohlenstoffatome.

Als Salze kommen Salze mit anorganischen und organischen Basen in Betracht. Als Kationen für die Salzbildung werden vor allem die Kationen der Alkalimetalle oder Erdalkalimetalle verwendet, es kommen jedoch auch die entsprechenden Kationen organischer Stickstoffbasen, wie Amine oder Aminoalkanole, Aminozucker etc. zur Anwendung. Beispielsweise seien die Salze von Natrium, Magnesium, Calcium, Dimethylamin, Diethylamin, Ethanolamin, Diethanolamin, Triethanolamin, Glucamin, N-Methylglucamin, Glucosamin, N-Methylglucosamin genannt.

Eine Ausgestaltung der Erfindung sind 6-Aryl-3[2H]pyridazinone der allgemeinen Formel Ia

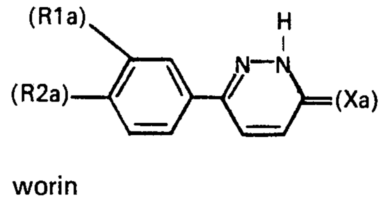

(Ia),

worin

R1a eine Methoxygruppe,

R2a eine Alkoxygruppe mit 2 bis 4 Kohlenstoffatomen oder eine Alkenyloxygruppe mit 3 oder 4 Kohlenstoffatomen und

Xa ein Sauerstoffatom bedeuten, und ihre pharmakologisch verträglichen Salze mit Basen.

Eine weitere Ausgestaltung der Erfindung sind 6-Aryl-3[2-H]pyridazinone der allgemeinen Formel Ib

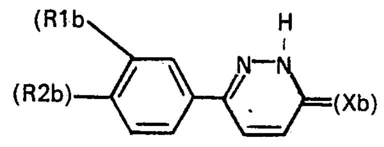

(Ib),

worin

R1b eine Alkoxygruppe mit 2 bis 4 Kohlenstoffatomen oder eine Alkenyloxygruppe mit 3 oder 4 Kohlenstoffatomen,

R2b eine Methoxygruppe und

Xb ein Sauerstoffatom bedeuten, und ihre pharmakologisch verträglichen Salze mit Basen.

Bevorzugte Vertreter der Ausgestaltung Ia sind solche, in denen R2a eine n-Propoxy-, Isopropoxy- oder Isobutoxygruppe darstellt. Besonders bevorzugte Vertreter sind die Verbindungen 6-(3-Methoxy-4-n-propoxyphenyl) -3[2H]pyridazinone und 6-(4-Isobutoxy-3-methoxyphenyl)-3[2H]pyridazinon.

Bevorzugte Vertreter der Ausgestaltung Ib sind solche, in denen R1b eine n-Propoxy-, Isopropoxy-, Allyl-oder Isobutoxygruppe darstellt. Besonders bevorzugte Vertreter sind die Verbindungen 6-(3-Isobutoxy- 4 -methoxyphenyl)-3[2H]pyridazinon und 6-(4-Methoxy-3-n-propoxyphenyl)-3-[2H]pyridazinon.

Ein weiterer Gegenstand der Erfindung ist die Verwendung der Verbindungen gemäss allgemeiner Formel I bei der Herstellung von Arzneimitteln zur Behandlung oder Prophylaxe von Krankheiten, die auf Erkrankungen der Bronchien und/oder einer Insuffizienz des Herzens beruhen, oder zur Stärkung des Herzens sowie die entsprechenden Arzneimittel.

Zur Verwendung bei der Herstellung von Arzneimitteln zur Behandlung oder Prophylaxe von

Krankheiten, die auf Erkrankungen der Bronchien beruhen, sind die Verbindungen 6-(3-Methoxy-4-n-propoxyphenyl)-3[2H] pyridazinon, 6-(4-Isobutoxy-3-methoxyphenyl)-3[2H]pyridazinon und 6- (4 -Methoxy- 3 -isopropoxyphenyl) -3[2H]pyridazinon bevorzugt, wobei die beiden erstgenannten Verbindungen besonders bevorzugt sind.

Zur Verwendung bei der Herstellung von Arzneimitteln zur Behandlung oder Prophylaxe von Krankheiten, die auf einer Insuffizienz des Herzens beruhen, oder zur Stärkung des Herzens sind die Verbindungen 6-(3-Isobutoxy-4-methoxyphenyl)-3-[2H]pyridazinon, 6-(4-Methoxy-3-n-propoxyphenyl)-3[2H]pyridazinon und 6-(3-Allyloxy-4-methoxyphenyl)-3[2H]pyridazinon bevorzugt, wobei die erstgenannte Verbindung besonders bevorzugt ist.

Gegenstand der Erfindung ist ferner ein Verfahren zur Herstellung der 6-Aryl-3[2H]pyridazinone der allgemeinen Formel I und ihrer pharmakologisch verträglichen Salze mit Basen, das dadurch gekennzeichnet ist, dass man

a) ein 6-Aryl-tetrahydropyridazinon der allgemeinen Formel II

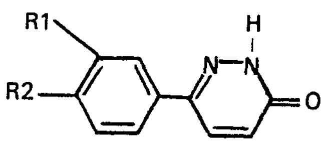

(II),

worin R1 und R2 die oben angegebene Bedeutung haben, oxydiert und gewünschtenfalls anschliessend das so erhaltene Pyridazinon I (X=O) in das Pyridazinthion I (X=S) und/oder in das Salz überführt, oder

b) eine Morpholinobuttersäure der allgemeinen Formel III

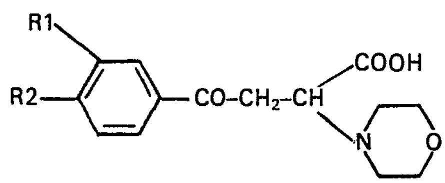

(III),

worin R1 und R2 die oben angegebene Bedeutung haben, mit Hydrazin umsetzt und gewünschtenfalls anschliessend das so erhaltene Pyridazinon I (X=O) in das Pyridazinonthion I (X=S) und/oder in das Salz überführt, oder

c) eine Acrylsäure der allgemeinen Formel IV

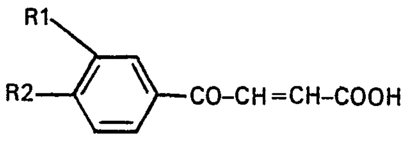

(IV),

worin R1 und R2 die oben angegebene Bedeutung haben, mit Hydrazin umsetzt und gewünschtenfalls anschliessend das so erhaltene Pyridazinon I (X=O) in das Pyridazinthion I (X=S) und/oder in das Salz überführt.

Die Oxydation (Dehydrierung) nach Verfahrensvariante a) erfolgt nach dem Fachmann bekannten Methoden. Beispielsweise kann die Dehydrierung in Gegenwart von Edelmetallen der 8. Nebengruppe, z.B. Palladium oder Platin [DE-OS 27 57 923]; mit Chromtrioxid [Overend et al. J. Chem. Soc. 1947, 239] mit Nitrobenzolsulfonsäure oder Nitronaphthalinsulfonsäuren, bevorzugt mit deren Natrium- oder Ammoniumsalzen [GB-PS 1 168 291] vorgenommen werden.

Die Umsetzung nach Verfahrensvariante b) erfolgt analog Schreiber et al. [Bull. Soc. chim. France 2(1973)625]. Beispielsweise wird die Morpholinobuttersäure III in einem Niederalkanol, z.B. n-Butanol, mit Hydrazinhydrat unter Rückfluss umgesetzt. Alternativ kann das durch Reaktion des entsprechenden Acetophenons mit Glyoxylsäure und Morpholin erhaltene Morpholiniumsalz der Verbindung III in saurer Lösung mit Hydrazinhydrat umgesetzt werden.

Die Umsetzung nach Verfahrensvariante c) erfolgt nach dem Fachmann bekannten Methoden. Beispielsweise werden die Verbindungen IV analog der DE-OS 24 45 681 in Gegenwart von basischen Verbindungen, wie Alkalimetallcarbonaten oder -hydroxiden oder -niederalkanolaten oder tert.-Aminen, mit Methanol oder wässrigem Methanol bei Raumtemperatur oder leicht erhöhter Temperatur umgesetzt, aus dem entstehenden Salz die Säure in Freiheit gesetzt und diese mit 1 bis 1,5 Mol Hydrazinhydrat erhitzt, wobei mindestens ein neutrales, vorzugsweise jedoch saures Milieu aufrechterhalten wird.

Die Überführung der erhaltenen Arylpyridazinone I (X= Sauerstoff) in die Arylpyridazinthione I (X=Schwefel) erfolgt nach dem Fachmann bekannten Methoden. Beispielsweise werden die Arylpyridazinone I analog Albright et al. [J. Heterocycl. Chem. 15(1978)881] mit Phosphoroxidhalogeniden bei 80–120 °C in Gegenwart eines Lösungsmittels, wie Toluol, Xylol, chlorierten Kohlenwasserstoffen, vorzugsweise ohne Lösungsmittel zu den entsprechenden 6-Aryl-3-halogenpyridazinen umgesetzt, deren Reaktion (Jahine et al. [Ind. J. Chem. 16B(1978)-1000–1003]) mit Thioharnstoff, z.B. durch 5–10stündiges Erhitzen in einem Niederalkanol, wie n-Butanol, Methyl- oder Ethylcellosolve bei 100 bis 140 °C, zu den Arylpyridazinthionen I führt.

Die Überführung der 6-Aryl-3 [2H]pyridazinone I in die Salze erfolgt nach dem Fachmann bekannten Methoden. Als alkalischer Reaktionspartner wird diejenige anorganische oder organische Base verwendet, deren Salz gewünscht wird. Man erhält die Salze beispielsweise, indem man die Pyridazinone I mit dem stöchiometrischen Äquivalent an entsprechender Base, z.B. Natriumhydroxid oder Natriummethanolat umsetzt, oder leicht lösliche Salze durch doppelte Umsetzung in schwer lösliche Salze überführt.

Für die Herstellung der neuen Verbindungen Ia und Ib werden entsprechende Ausgangsverbindungen IIa, IIb, IIIa, IIIb, IVa oder IVb

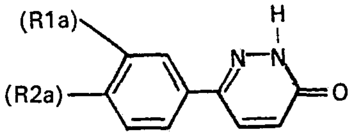

(IIa),

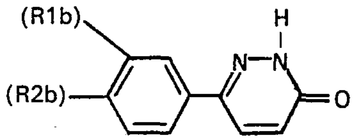

(IIb),

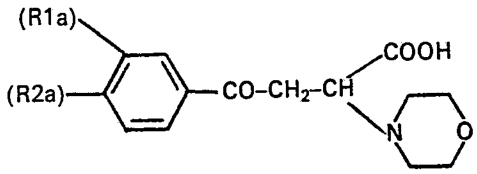

(IIIa),

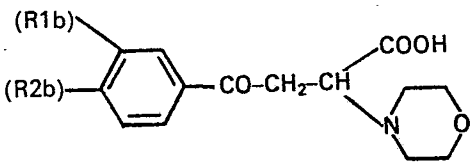

(IIIb),

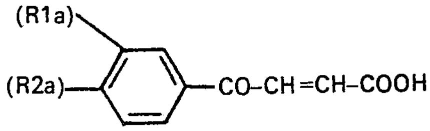

(IVa),

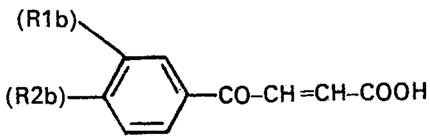

(IVb),

worin R1a, R1b, R2a und R2b die oben angegebene Bedeutung haben, eingesetzt.

Die Verbindungen II, III und IV sind bekannt oder können nach bekannten Verfahren hergestellt werden.

Die folgenden Beispiele dienen zur näheren Erläuterung der Erfindung. Fp. bedeutet Schmelzpunkt, Temperaturangaben erfolgen in °C.

Beispiel 1

6-(4-n-Butoxy- 3 -methoxyphenyl)-3[2H]pyridazinon 11,0 g 6-(4-Hydroxy-3-methoxyphenyl)-4,5-dihydro-3[2H] pyridazinon werden zusammen mit 8,2 g 1-Brombutan und 8,3 g Kaliumcarbonat in 150 ml wasserfreiem Aceton 20 Stunden am Rückfluss erhitzt. Danach lässt sich dünnschichtchromatographisch die Ausgangsverbindung nicht mehr nachweisen. Die Suspension wird heiss filtriert, der Filterkuchen wird mit heissem Aceton ausgewaschen, die Filtrate werden vereinigt und im Vakuum eingedampft. Der halbfeste Rückstand [6-(4-n-Butoxy-3-methoxyphenyl)-4,5-dihydro-3[2H]pyridazinon] wird in 50 ml Ethanol aufgenommen, mit 10,0 g Ätznatron in 200 ml Wasser und 14,6 g Natrium-meta-nitrobenzolsulfonat versetzt und 2 Stunden am Rückfluss erhitzt. Nach dem Abkühlen wird mit konzentrierter Salzsäure auf pH 1–2 angesäuert, der kristalline Feststoff wird abgesaugt, die Lösung wird mehrmals mit Chloroform extrahiert. Der Rückstand aus dem Chloroformextrakt wird mit den Kristallen vereinigt und aus Ethanol/Essigsäureethylester umkristallisiert. Man erhält 8,7 g (63,7% d.Th.) der Titelverbindung vom Fp. 193°.

Analog erhält man

6-(4-Allyloxy- 3 -methoxyphenyl)-3[2H]pyridazinon, Fp. 165°; durch Umsetzung von 6-(4-Hydroxy-3-methoxyphenyl)-4,5-dihydro-3[2H]pyridazinon mit Allylbromid und Oxydation des erhaltenen 4,5-Dihydro-3[2H]pyridazinons.

Beispiel 2

6-(4-Ethoxy-3-methoxyphenyl)-3[2H]pyridazinon

a) 4,5 g 4-(4-Ethoxy-3-methoxyphenyl)-2-morpholino-4-oxobuttersäure werden in 30 ml 1-Butanol mit 6,7 g 100%igem Hydrazinhydrat 8 Stunden am Rückfluss gekocht. Anschliessend wird das Reaktionsgemisch im Vakuum eingedampft; der Rückstand wird in 100 ml 2 N Salzsäure aufgekocht, abgesaugt und mit Wasser säurefrei gewaschen. Nach Trocknung erhält man 2,1 g (65,6% d.Th.) der Titelverbindung vom Fp. 182–184°, die nach dem Umkristallisieren aus Ethanol/Essigsäureethylester bei 186° schmilzt.

Analog erhält man

6-(4-Allyloxy-3-methoxyphenyl)-3[2H]pyridazinon, Fp. 165°;

6-(3-Methoxy-4-n-propoxyphenyl)-3[2H]pyridazinon, Fp. 172°;

6-(4-n-Butoxy-3-methoxyphenyl)-3[2H]pyridazinon, Fp. 193°;

6-(4-Ethoxy-3-methoxyphenyl)-3[2H]pyridazinon, Fp. 192°; durch Umsetzung der entsprechenden 4-Aryl-2-morpholino-4-oxobuttersäuren mit Hydrazinhydrat.

b) die verwendeten 4-Aryl-2-morpholino-4-oxobuttersäuren werden wie folgt hergestellt:

8,7 g Glyoxylsäuremonohydrat werden in 50 ml Ethanol bis zur vollständigen Lösung aufgekocht, dann werden 16,5 g Morpholin und 18,5 g 4-Ethoxy-3-methoxyacetophenon zugefügt und die Mischung 16 Stunden bei 50° gerührt. Die Reaktionsmischung wird im Vakuum eingedampft, der Rückstand wird mit Aceton digeriert, gekühlt, abgesaugt und im Vakuum getrocknet. Man erhält, 9,9 g Morpholinium-4-(4-ethoxy-3-methoxyphenyl)-2-morpholino-4-oxobutyrat vom Fp. 131°. Zur Freisetzung der Säure wird das Morpholiniumsalz in 50 ml Wasser gelöst und mit Essigsäure auf pH 4,5 angesäuert; die ölig anfallende Säure wird mit Chloroform extrahiert, der Chloroformextrakt wird über Natriumsulfat getrocknet und eingedampft. Der Rückstand wird mit Essigsäureethylester digeriert, abgesaugt und im Vakuum getrocknet. Man erhält 4,9 g der freien Säure vom Schmp. 157°.

Analog werden

Morpholinium-4-(3-methoxy-4-n-propoxyphenyl)-2-morpholino-4-oxobutyrat, Fp. 110°;

Morpholinium-4-(4-n-butoxy-3-methoxyphenyl)-2-morpholino-4-oxobutyrat, Fp. 120 °;
4-(4-Allyloxy-3-methoxyphenyl)-2-morpholino-4-oxobuttersäure, Fp. 166°;
Morpholinium-4-(4-ethoxy-3-methoxyphenyl)-2-morpholino-4-oxobutyrat, Fp. 131°;
4-(4-Ethoxy-3-methoxyphenyl)-2-morpholino-4-oxobuttersäure, Fp. 157° aus den entsprechenden Acetophenonen hergestellt.

Beispiel 3

6-(3-Methoxy-4-n-propoxyphenyl)-3[2H]-pyridazinon

a) 10,6 g 3-(3-Methoxy-4-n-propoxybenzoyl)-acrylsäure und 6,1 g Kaliumcarbonat werden in 100 ml Methanol gelöst und über Nacht bei Raumtemperatur gerührt. Man säuert mit 7,4 ml konzentrierter Salzsäure an, setzt dem Gemisch 2,2 g 100%iges Hydrazinhydrat zu und kocht 3 Stunden am Rückfluss. Dann wird mit 1 ml Salzsäure auf pH 1 angesäuert, im Verlaufe von 2 Stunden werden 75 ml Methanol abdestilliert. Man lässt abkühlen, saugt die Kristallmasse ab, schlämmt sie mehrmals mit je 50 ml Wasser auf, saugt ab und trocknet sie schliesslich im Vakuum. Man erhält 6,6 g (63,7% d.Th.) der Titelverbindung vom Fp. 169–171°. Nach Umkristallisation aus Ethanol/Essigsäureethylester steigt der Fp. auf 172°.

Analog erhält man
6-(4-n-Butoxy-3-methoxyphenyl)-3[2H]pyridazinon, Fp. 193°;
6-(4-Ethoxy-3-methoxyphenyl)-3 [2H]pyridazinon, Fp. 186°;
6-(4-sec.-Butoxy-3-methoxyphenyl)-3[2H] pyridazinon, Fp. 168°;
6-[4-(3-Methylbutoxy)-3-methoxyphenyl)-3-[2H] pyridazinon, Fp. 170°; aus den entsprechend substituierten 3-Benzoylacrylsäuren.

b) Die Benzoylacrylsäuren werden wie folgt hergestellt:

10,4 g (3-Methoxy-4-n-propoxy)-acetophenon werden mit 9,2 g Glyoxylsäuremonohydrat gemischt und im Ölbad 1 Stunde auf 110° erhitzt. Anschliessend wird das Gemisch mit 200 ml Wasser aufgekocht, abgekühlt und mit Chloroform extrahiert. Zur besseren Phasentrennung wird die wässrige Phase mit Kochsalz gesättigt. Nach Trocknung des Chloroformextrakts über Natriumsulfat und Eindampfen im Vakuum wird der orangerote Rückstand aus Aceton kristallisiert. Man erhält 10,4 g (78,7% d.Th.) 3-(3-Methoxy-4-n-propoxybenzoyl)-acrylsäure vom Fp 158°.

Analog erhält man
3-(4-n-Butoxy-3-methoxybenzoyl)-acrylsäure, Fp. 136°; 92,5% d.Th.,
3-(4-Ethoxy-3.Methoxybenzoyl)-acrylsäure, Fp. 154°, 95,3% d.Th.,
3-(4-Methoxy-3-n-propoxybenzoyl)-acrylsäure,
3-(3-Ethoxy-4-methoxybenzoyl)-acrylsäure,
3-(3-Allyloxy-4-methoxybenzoyl)-acrylsäure,
3-[3-(3-Methylbutoxy)-4-methoxybenzoyl]-acrylsäure,
3-(4-sec.-Butoxy-3-methoxybenzoyl)-acrylsäure, Fp. 128°; 96% d.Th.,
3-[4-(3-Methylbutoxy)-3-methoxybenzoyl]-acrylsäure, Fp. 110°; 93,1% d.Th. aus den entsprechenden substituierten Acetophenonen.

c) Die substituierten Acetophenone werden wie folgt hergestellt:

50 g 4-Hydroxy-3-methoxyacetophenon werden in 100 ml Dimethylformamid gelöst, mit 94 g 80%iger Natriumhydrid-Mineralöl-Suspension portionsweise versetzt, der Lösung werden nach beendeter Wasserstoffentwicklung 40,7 g n-Propylbromid, gelöst in 200 ml Dimethylformamid, zugefügt. Die Lösung wird 2 Stunden bei 100° gerührt und im Vakuum vom Lösungsmittel befreit; Der ölige Rückstand wird zwischen 2 N Natronlauge und Dichlormethan verteilt und die wässrige Phase wird noch zweimal mit Dichlormethan extrahiert. Nach Trocknung über Kaliumcarbonat werden die vereinigten organischen Extrakte im Vakuum eingedampft, der Rückstand wird aus Cyclohexan kristallisiert. Man erhält 50,6 g (80,8% d.Th.) 3-Methoxy-4-n-propoxyacetophenon vom Fp. 42°.

Analog erhält man
4-N-Butoxy-3-methoxyacetophenon, Fp. 45 °, 82% d.Th.;
4-Ethoxy-3-methoxyacetophenon, Fp. 76°, 90,8% d.Th.;
4-Allyloxy-3-methoxyacetophenon, Sdp. 125°/0,133 mbar, 92,3% d.Th.;
4-Isopropyloxy-3-methoxyacetophenon, Fp. 111°, 79,7% d.Th.;
4-sec.-Butyloxy-3-methoxyacetophenon, Öl, 95,5%, d.Th.;
4-(3-Methylbutoxy)-3-methoxyacetophenon, Öl, 95,8% d.Th.,
4-Methoxy-3-n-propoxyacetophenon, Fp. 71°, 68,7% d.Th.;
3-Ethoxy-4-methoxyacetophenon, Fp. 67°, 74,3% d.Th.;
3-Allyloxy-4-methoxyacetophenon, Fp. 49°, 77% d.Th.;
3-Isopropyloxy-4-methoxyacetophenon, Fp. 36°, 75,7% d.Th.;
3-(3-Methylbutoxy)-4-methoxyacetophenon, Öl, 65% d.Th.;
3-Isobutoxy-4-methoxyacetophenon, Fp. 69°, 91,8% d.Th.;
4-Isobutoxy-3-methoxyacetophenon, Fp. 38°, 91,8% d.Th.

Beispiel 4

6-(3-Methoxy-4-n-propoxyphenyl)-3[2H]pyridazinthion

a) 13,9 g 3-Chlor-6-(3-Methoxy-4-n-propoxyphenyl)-pyridazin werden mit 5,5 g Thioharnstoff in 50 ml Ethylenglykolmonomethylether 8 Stunden am Rückfluss gekocht. Die Mischung wird nach dem Abkühlen mit 250 ml Wasser verdünnt und mit Chloroform dreimal extrahiert. Die Choroformextrakte werden über Natriumsulfat getrocknet und eingedampft. Der Rückstand wird aus Ethanol/Essigsäureethylester kristallisiert. Man erhält 4,8 g (34,6% d.Th) der Titelverbindung vom Fp. 174–176°.

Analog erhält man:
6-(3-Ethoxy-4-methoxyphenyl)-3[2H]pyridazinthion, Fp. 183°, 88,9% d.Th.;
6-(3-Isopropoxy-4-methoxyphenyl)-3[2H]-pyridazinthion, Fp. 168°, 93,4% d.Th.;
6-(3-Isobutoxy-4-methoxyphenyl)-3[2H)pyridazinthion, Fp. 170°, 66,7% d.Th.;
6-(4-Isobutoxy-3-methoxyphenyl)-3[2H]pyridazinthion, Fp. 145°, 100% d.Th.

b) 29,9 g 6-(3-Methoxy-4-n-propoxyphenyl)-3[2H]pyridazinon werden portionsweise in 65 ml Phosphoroxidtrichlorid unter Rühren eingetragen und anschliessend 1 Stunde bei 100° gerührt. Die Reaktionsmischung wird im Vakuum auf die Hälfte eingeengt und unter gutem Rühren auf Eis gegeben. Die sich abscheidenden Kristalle werden abgesaugt, mit Wasser gewaschen und im Vakuum getrocknet. Man erhält 31,6 g 3-Chlor-6-(3-methoxy-4-n-propoxyphenyl)-pyridazin, Fp. 129°; 98,7% d.Th.

Analog erhält man:
3-Chlor-6-(3-ethoxy-4-methoxyphenyl)-pyridazin, Fp. 141°, 63,9% d.Th.,
3-Chlor-6-(3-isopropoxy-4-methoxyphenyl)-pyridazin, Fp. 144°, 94,9% d.Th.;
3-Chlor-6-(3-isobutoxy-4-methoxyphenyl)-pyridazin, Fp. 137°, 96,1% d.Th.;
3-Chlor-6-(4-isobutoxy-3-methoxyphenyl)-pyridazin, Fp. 116°, 95,8% d.Th.

Beispiel 5

6-(3-Methoxy-4-n-propoxyphenyl)-3[2H]-pyridazinon-Natriumsalz

26 g 6-(3-Methoxy-4-n-propoxyphenyl)-3[2H]pyridazinon werden in 150 ml Methanol mit 60 g Natriummethylat (97%ig) 30 Minuten bei 50° gerührt und anschliessend im Vakuum zur Trockne eingedampft. Der farblose Rückstand wird mit Ethanol ausgekocht. Man erhält nach Trocknung 24,8 g (87,9 % d.Th) als farblosen Feststoff, der bis 280° nicht schmilzt.

Beispiel 6

6-(3-Isopropoxy-4-methoxyphenyl)-3-[2H]-pyridazinon

9,1 g 3-Isopropoxy-4-methoxyacetophenon werden mit 4,0 g Glyoxylsäuremonohydrat gemischt und 1,5 Stunden auf 110° erhitzt. Nach dem Abkühlen auf 50° wird die Schmelze mit 30 ml Wasser verdünnt und mit 10 ml konzentrierter, wässriger Ammoniaklösung alkalisch gestellt. Nach Zugabe von 2,2 g Hydrazinhydrat wird die Lösung 2 Stunden am Rückfluss gekocht, wobei sich die Titelverbindung kristallin abscheidet. Nach dem Abkühlen der Suspension wird diese abgesaugt, der Filterkuchen mit Wasser neutral gewaschen und im Vakuum getrocknet. Nach Umkristallisation aus Essigester erhält man 8,5 g (75,2% d.Th.) der Titelverbindung vom Fp. 160°.

Analog erhält man
6-(4-Isobutoxy-3-methoxyphenyl)-3[2H]pyridazinon, Fp. 184°, 42,2% d.Th.;
6-(3-Isobutoxy-4-methoxyphenyl)-3-[2H]pyridazinon, Fp. 186°, 58,4% d.Th.,
6-(3-Ethoxy-4-methoxyphenyl)-3 [2H]pyridazinon, Fp. 171°, 73,1% d.Th.,
6-(3-Methoxy-4-n-propoxyphenyl)-3-[2H]pyridazinon, Fp. 173°, 66,3% d.Th.

Beispiel 7

6-[4-Methoxy-3-(3-methylbutoxy)-phenyl]-3[2H]pyridazinon

18 g 4-Methoxy-3-(3-methylbutoxy)-acetophenon und 7,7 g Glyoxylsäuremonohydrat werden gemischt und 1,5 Stunden auf 110° erhitzt. Nach dem Abkühlen wird die Schmelze in 50 ml Methanol gelöst, mit 11,6 g Kaliumcarbonat versetzt und über Nacht bei 20–25° gerührt. Anschliessend wird das Gemisch mit 84 ml 1 N Salzsäure neutralisiert, mit 4,2 g Hydrazinhydrat versetzt und 3 Stunden am Rückfluss gekocht. Schliesslich wird mit 20 ml konzentrierter Salzsäure bis auf pH 1 angesäuert, nochmals kurz aufgekocht und die Reaktionsmischung im Vakuum weitgehend vom Methanol befreit. Das teils ölig, teils kristallin anfallende Reaktionsprodukt wird mit Chloroform extrahiert, die vereinigten Extrakte über Natriumsulfat getrocknet und im Vakuum eingedampft. Nach Umkristallisation des Rückstandes aus Isopropanol erhält man 9,6 g (43,8% d.Th.) der Titelverbindung vom Fp. 192°.

Analog erhält man
6-(4-Methoxy-3-n-propoxyphenyl)-3[2H]pyridazinon, Fp. 196°, 17,5% d.Th).,
6-(3-Allyloxy-4-methoxyphenyl)-3[2H]pyridazinon, Fp. 175°, 16,3% d.Th.,
6-(3-Ethoxy-4-methoxyphenyl)-3[2H]pyridazinon, Fp. 171°, 15,1 % d.Th.,
6-(4-Isopropoxy-3-methoxyphenyl)-3[2H]pyridazinon, Fp. 200 °, 26,4% d.Th.

Gewerbliche Anwendbarkeit

Die 6-Aryl-3[2H]pyridazinone der allgemeinen Formel I und die der Ausgestaltungen Ia oder Ib besitzen wertvolle Eigenschaften, die sie gewerblich verwertbar machen. Sie zeichnen sich überraschenderweise durch eine bronchospasmolytische und/oder cardiotonische Wirkung aus, die diejenige des Theophyllin bzw. des Theophyllin-Ethylendiamin z.T. erheblich übertrifft. Ausserdem weisen sie im Vergleich zu 6-(4-Methoxyphenyl)-3 [2H]pyridazinon eine stärkere bronchospasmolytische bzw. positiv inotrope Wirkung auf.

Die bronchospasmolytische Wirksamkeit der 6-Aryl-3[2H]pyridazinone ermöglicht ihren Einsatz in der Human- und Veterinärmedizin, wobei sie zur Behandlung und Prophylaxe von Krankheiten, die auf Erkrankungen der Bronchien beruhen, verwendet werden. Beispielsweise können chronisch obstruktive Atemwegserkrankungen verschiedener Genese (Bronchitis, Asthma bronchiale) bei Mensch und Tier behandelt werden.

Die positiv inotrope Wirksamkeit der 6-Aryl-3[2H]pyridazinone ermöglicht ihren Einsatz in der Human- oder Veterinärmedizin, wobei sie zur Behandlung von Krankheiten, die auf einer Insuffizienz des Herzens beruhen, oder zur Stärkung des Herzens verwendet werden. Beispielsweise

werden Herzmuskelschwäche, Herzinsuffizienz Altersherz, Myokardinfarkt, Herzkreislaufschwäche, Stenokardie bei mangelnder Herzleistung und Koronarinsuffizienz bei Mensch und Tier behandelt.

Ein weiterer Gegenstand der Erfindung sind Arzneimittel, die ein oder mehrere der 6-Aryl-3[2H]pyridazinone der allgemeinen Formel I oder der Ausgestaltungen Ia oder Ib enthalten.

Die Arzneimittel werden nach an sich bekannten Verfahren hergestellt, wobei die Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, so beträgt der Wirkstoffgehalt dieser Mischungen 0,5 bis 95, vorzugsweise 15 bis 75 Gewichtsprozent der Gesamtmischung.

Die Wirkstoffe bzw. die Arzneimittel werden in jeder geeigneten Formulierung angewandt unter der Voraussetzung, dass die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Üblicherweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion oder einer Suspension sein.

Unter «Einheitsdosis» im Sinne der Erfindung wird eine physikalisch bestimmte Einheit, die eine individuelle Menge das aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäss der Erfindung können, wenn sie in Einheitsdosen vorliegen und für die Applikation z.B. am Menschen bestimmt sind, etwa 5 bis 250 mg, vorteilhafterweise 10 bis 200 mg und insbesondere 20 bis 100 mg Wirkstoff enthalten. Parenterale Zubereitungen können etwa 1 bis 50 mg, vorteilhafterweise 3 bis 30 mg und insbesondere 5 bis 25 mg Wirkstoff enthalten.

Im allgemeinen werden in der Humanmedizin der oder die Wirkstoffe bei oraler Gabe in einer Tagesdosis von 0,1 bis 10, vorzugsweise 0,3 bis 5, insbesondere 0,5 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 0,2 bis 3, insbesondere 0,4 bis 2 mg/kg Körpergewicht. Für die inhalative Verabreichung ist es vorteilhaft, den oder die Wirkstoffe in einer Tagesdosis von 0,1 bis 10 mg, vorzugsweise 0,5 bis 5 mg, insbesondere 1 bis 3 mg, gegebenenfalls in Form mehrerer, vorzugsweise 1 bis 3 Einzelgaben zu verabreichen.

Zubereitungen für die intravenöse Verabreichung sind vor allem für die akute Behandlung, z.B. Notfallbehandlung, zweckmässig.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Bei akuten Fällen wird zu Beginn der Behandlung eine höhere Dosis verabreicht. Nach Eintreten der gewünschten Wirkung wird auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens erfolgen.

Die pharmazeutischen Zubereitungen bestehen in der Regel aus den erfindungsgemässen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler, für die Arzneimittelaufnahme durch den Körper, als Formulierungshilfsmittel, als Süssungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumsphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium oder Magnesiumstearat, Talkum oder Silikonöl, enthalten. Sie können zusätzlich mit einem Überzug versehen sein, der auch so beschaffen sein kann, dass

er eine verzögerte Auflösung und Resorption des Arzneimittels im Gastrointestinaltrakt bewirkt, so dass z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen , z.B. Oliven-, Erdnuss- oder Paraffinöl, Verdünnungsmittel enthalten.

Wässrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süssungsmittel, z.B. Saccharose, Lactose, Natriumcyclamat, Dextrose, Invertzuckersirup, enthalten.

Ölige Suspensionen können z.B. Erdnuss-, Oliven-, Sesam-, Kokos- oder Paraffinöl und Verdikkungsmittel, wie z.B. Bienenwachs, Hartparaffin oder Cetylalkohol, enthalten; ferner Süssungsmittel, Geschmacksmittel und Antioxidantien.

Im Wasser dispergierbare Pulver und Granulate können die Arzneistoffe in Mischungen mit Dispergier-, Benetzungs- und Suspendiermitteln, z.B. den oben genannten, sowie mit Süssungsmitteln, Geschmacksmitteln und Farbstoffen, enthalten.

Emulsionen können z.B. Oliven-, Erdnuss- oder Paraffinöl neben Emulgiermitteln, wie z.B. Akaziengummi, Traganthgummi, Phosphatiden, Sorbitanmonooleat, Polyoxyethylensorbitanmonooleat, und Süssungs- und Geschmacksmitteln enthalten.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare, gegebenenfalls kurzfristig herzustellende, wässrige Suspensionen, isotonische Salzlösungen oder sonstige Lösungen, die Dispergier- oder Benetzungsmittel und/oder pharmakologisch verträgliche Verdünnungsmittel z.B. Propylen- oder Butylenglykol, enthalten.

Die orale Anwendung der Arzneistoffe ist bevorzugt.

Bevorzugt für die Verwendung als Bronchospasmolytikum ist auch die inhalative Applikation der erfindungsgemässen Verbindungen. Diese werden entweder direkt als Pulver oder durch Vernebeln von die erfindungsgemässen Verbindungen enthaltenden Lösungen oder Suspensionen verabreicht. Die Vernebelung kann dabei auf herkömmliche Weise, beispielsweise durch Pressluftvernebler oder Ultraschallvernebler, erfolgen. Besonders vorteilhaft ist die Verabreichung aus Sprühdosen, insbesondere solcher mit einem herkömmlichen Dosierventil (Dosier-Aerosole). Mittels Dosier-Aerosolen ist es möglich, pro Sprühstoss eine definierte Menge an Wirkstoff bereitzustellen. Von besonderem Vorteil sind hier sogenannte Synchron-Inhalatoren, womit die Wirkstoffabgabe synchron zur Einatmung geschehen kann. Geeignete Synchron-Inhalationsvorrichtungen sind z.B. in der DE-PS 19 45 257, DE-PS 19 17 911 und DE-OS 20 55 734 offenbart.

Für Inhalationszwecke kommen die Wirkstoffe vorzugsweise in mikronisierter Form zum Einsatz, wobei Teilchengrössen von weniger als 10 µm vorteilhaft sind. Zur Applikation aus Sprühdosen werden die Wirkstoffe in üblichen Treibmitteln dispergiert, vorzugsweise unter Zuhilfenahme eines Dispergiermittels. Als Treibmittel kommen insbesondere Gemische von Trichlorfluormethan (Frigen® 11) und Dichlordifluormethan (Frigen® 12) in Frage, wobei Trichlorfluormethan ganz oder teilweise durch 1,1,2-Trichlortrifluorethan (Frigen® 113) ersetzt werden kann. Als Dispergiermittel kommen insbesondere die für diese Zwecke gebräuchlichen Sorbitanester (Spane® der Firma Atlas GmbH) und Lecithin in Frage. Das Dispergiermittel wird in der gekült vorgelegten schwerer flüchtigen Treibmittelkomponente gelöst. In der Lösung wird der mikronisierte Wirkstoff bzw. werden die mikronisierten Wirkstoffe eingerührt. Die Dispersion wird in Sprühbüchsen eingefüllt. Nach dem Vercrimpen wird die leichter flüchtige Treibmittelkomponente aufgedrückt.

Der oder die Wirkstoffe können gegebenenfalls mit einem oder mehreren angegebenen Träger- oder Zusatzstoffe auch in mikroverkapselter Form formuliert werden.

Tabletten mit 100 mg 6-(4-Isopropoxy-3-methoxyphenyl)-3[2H] pyridazinon

40 kg Wirkstoff, 24 kg Milchzucker und 16 kg Maisstärke werden mit 4 kg Polyvinylpyrrolidon (MG ~ 25 000) in 5,5, Liter Wasser granuliert und durch ein Sieb von 1,25 mm Maschenweite gepresst. Nach dem Trocknen werden 10 kg Carboxymethylcellulose, 4 kg Talkum und 2 kg Magnesiumstearat zugegeben. Auf einer Exzentermaschine wird das Granulat zu Tabletten von 9 mm Durchmesser, 250 mg Gewicht und einer Härte von 4 bis 5 kg verpresst.

Kapseln mit 15 mg 6-(3-Methoxy-4-n-propoxyphenyl)-3[2H]pyridazinon

150 mg Wirkstoff, 845 mg mikrokristalline Cellulose und 5 mg amorphe Kieselsäure werden feingepulvert, gut vermischt und in Hargelatinekapseln Grösse 4 abgefüllt.

Dosieraerosolzubereitung enthaltend 6-(4-Isobutoxy-3-methoxyphenyl)-3[2H] pyridazinon

0,540 g Span® 85 und 0,135 g Aroma werden in 10,215 g gekühltem Frigen® 11 gelöst. In die Lösung werden 0,270 g mikronisierter Wirkstoff eingerührt und in 24 ml-Dosen eingefüllt. Nach dem Vercrimpen werden 14,971 g Frigen® 12 eingepresst. Bei einem Kammervolumen des Dosierventils von 125 µl werden pro Ventilhub 1,6 mg Wirkstoff als Aerosol freigesetzt.

Biologische Untersuchungen

Die 6-Aryl-3[2H]pyridazinone der allgemeinen Formel I weisen eine bronchospasmolytische und/oder cardiotonische Wirkung auf, welche diejenige des Theophyllin bzw. des Theophyllin-Ethylendiamin z.T. erheblich übertrifft. Ausserdem weisen sie im Vergleich zu 6-(4-Methoxyphenyl)-3[2H]pyridazinon eine stärkere bronchospasmolytische bzw. positive inotrope Wirkung auf, wie der Vergleich an bekannten Versuchsanordnungen erkennen lässt.

Die relaxierende Wirkung der 6-Aryl-3[2H]pyridazinone I wurde an der Trachealspangenkette (Tr.) des Meerschweinchens in vitro geprüft. Die positiv inotrope Wirkung wurde am linken, elektrisch gereizten Vorhof der Ratte (li.V.) in vitro geprüft. Als Mass für die organselektive Wirksamkeit dient der Quotient aus $[EC_{40pot.}]_{linker\ Vorhof}$ und $[EC_{50}]_{Trachea}$. Als Mass für die Wirksamkeit werden die Quotienten der $[EC_{50}]_{Trachea}$-Werte bzw. $[EC_{40pot.}]_{linker\ Vorhof}$-Werte von Theophyllin und geprüfter Verbindung angegeben.

Die Verbindungen werden in den folgenden Tabellen durch eine laufende Nummer bezeichnet:

1: 6-(4-Isopropoxy-3-methoxyphenyl-3[2H]-pyridazinon
2: 6-(3-Methoxy-4-n-propoxyphenyl)-3[2H]-pyridazinon
3: 6-(4-n-Butoxy-3-methoxyphenyl)-3[2H]-pyridazinon
4: 6-(4-Ethoxy-3-methoxyphenyl)-3[2H]-pyridazinon
5: 6-(4-Allyloxy-3-methoxyphenyl)-3-[2H]pyridazinon
6: 6-[3-Methoxy-4-(3-methylbutoxy)-phenyl]-3-[2H]pyridazinon
7: 6-(4-sec-Butoxy-3-methoxyphenyl)-3[2H]-pyridazinon
8: 6-(3-Ethoxy-4-methoxyphenyl)-3[2H]pyridazinon
9: 6-(3-Allyloxy-4-methoxyphenyl)-3[2H]pyridazinon
10: 6-(4-Methoxy-3-n-propoxyphenyl)-3[2H]-pyridazinon
11: 6-(4-Methoxy-3-isopropoxyphenyl)-3[2H]-pyridazinon
12: 6-[4-Methoxy-3-(3-methylbutoxy)-phenyl]-3[2H]pyridazinon
13: 6-(3-Methoxy-4-n-propoxyphenyl)-3[2H]-pyridazinthion
14: 6-(3-Ethoxy-4-methoxyphenyl)-3[2H]-pyridazinthion
15: 6-(4-Methoxy-3-isobutoxyphenyl)-3[2]-pyridazinon
16: 6-(3-Methoxy-4-isobutoxyphenyl-3[2H]-pyridazinon
17: 6-(4-Methoxy-3-isopropoxyphenyl)-3[2H]-pyridazinthion
18: 6-(3-Isobutoxy-4-methoxyphenyl)-3[2H]-pyridazinthion
19: 6-(4-Methoxyphenyl)-3[2H]pyridazinon

*Tabelle I:* Bronchospasmolytische und positiv inotrope Wirkung, Toxizität

| lfd Nr. | A | B | C | D | E | F |
|---|---|---|---|---|---|---|
| 1 | 5,72 | 4,65 | 11,8 | 70,8 | 18,6 | 270 |
| 2 | 6,38 | 5,14 | 17,4 | 324 | 57,6 | 530 |
| 3 | 5,60 | 5,18 | 2,6 | 53,8 | 63,2 | >761* |
| 4 | 5,88 | 5,42 | 2,9 | 102 | 109 | |
| 5 | 5,82 | 5,18 | 4,4 | 85,2 | 63,1 | |
| 6 | 6,15 | 4,04 | 219 | 190 | 4,6 | >800* |
| 7 | 5,88 | 5,46 | 2,6 | 102 | 120 | |
| 8 | 6,47 | 5,88 | 3,9 | 398 | 316 | 550 |
| 9 | 6, 27 | 5,90 | 2,3 | 251 | 331 | 610 |
| 10 | 6,33 | 6,20 | 1,3 | 288 | 661 | 770 |
| 11 | 7,01 | <3,0 | 10 000 | 1 370 | – | 600 |
| 12 | 6,30 | <3,0 | 2 000 | 269 | – | 960 |
| 13 | 5,51 | <3,0 | 324 | 43,7 | – | |
| 14 | 5,85 | 5,68 | 1,5 | 95,5 | 200 | |
| 15 | 6,14 | 6,43 | 0,5 | 186 | 1 122 | |
| 16 | 6,54 | 5,78 | 5,8 | 468 | 251 | |
| 17 | 5,14 | 5,56 | 0,4 | 18,6 | 151 | – |
| 18 | 5,39 | 5,60 | 0,6 | 33,1 | 166 | – |
| 19 | 5,05 | 3,77 | 19,1 | 15,1 | 4,7 | >400* |
| Theophyllin | 3,87 | 3,38 | 3,1 | 1 | 1 | 280 |

Spalte A: -lg $[EC_{50}]_{Trachea}$
Spalte B: -lg$[EC_{40pot.}]_{linker\ Vorhof}$
Spalte C: $[EC_{40pot}1.]/[EC_{50}]$
Spalte D: $[EC_{50}]_{Theophyllin}[EC_{50}]_{Substanz}$
Spalte E: $[EC_{40pot.}]_{Theophyllin}[EC_{40pot.}]$Substanz
Spalte F: Circa-$LD_{50}$, Maus p.o., Werte mit * sind Angaben der Dosis tolerata

Die bronchospasmolytische Wirkung der Verbindungen auf die Trachealspangen-Kette des Meerschweinchens wurde in vitro geprüft: Vier parallele, aus jeweils 6 Einzelringen bestehende Trachealspangen-Ketten des Meerschweinchens (♂ und ♀, 430–600 g) im Organbad [ 5 ml, Krebs-Henseleit-Lösung mit Zusatz von Phentolamin ($10^{-5}$ mol/l), 37 °C, Vorspannung der Organe 2 g, Begasung mit Carbogen] entwickeln nach etwa 20 bis 30 Munuten eine stabile, tonische Spontankontraktur. An diesen dauerkontrahierten Organen kann unter isometrischen Messbedingungen durch Applikation der Prüfsubstanz in kumulativ-halblogarithmisch ansteigender Konzentration (z.B. $1 \times 10^{-6} + 2 \times 10^{-6} + 7 \times 10^{-6} + 2 \times 10^{-5}$ usw. mol/l) eine Relaxation herbeigeführt werden, wobei nach jeder Einzeldosis der Testsubstanz eine konstante Relaxations-Antwort abgewartet wird, bevor die nächsthöhere Konzentration appliziert wird. Über einen Zeitraum von 20 bis 30 Minuten wird somit eine vollständige Dosis-Wirkungskurve der Testsubstanz erhalten. Die jeweilige Relaxation wird als Prozentbruchteil der durch Gabe von (–)Isoprenalin ($10^{-6}$ mol/l) maximal erreichbaren Relaxation ausgedrückt. Als Mass für die bronchodilatorische Aktivität dient die Konzentration der Testsubstanz, welche 50% der maximal erreichbaren Relaxation bewirkt, ausgedrückt durch den negativen Logarithmus der $EC_{50}$ mol/l: –lg$[EC_{50}]$.

Die positiv inotrope Wirkung der Verbindungen wurde am linken, elektrisch gereizten Vorhof der Ratte in vitro geprüft. Registriert wurden isometrische Kontraktionen (HSE-Kraftaufnehmer K-30; Watanabe-Schreiber Linear Corder Mark 5) isolierter, linker Vorhöfe von Ratten (♂, 250–300 g) im Organbad (10 ml, Tyrode-Nährlösung, 31 °C, Begasung mit Carbogen, Vorspannung der Organe 0,25 g) unter elektrischer Stimulation (HSE-Reizgerät, 7 V, 3 ms, 2Hz). Nach einer Äquilibrierungszeit von 30 min kann durch Applikation der Prüfsubstanz in kumulativ-halblogarithmisch ansteigender Konzentration (z.B. $1 \times 10^{-6} + 2 \times 10^{-6} + 7 \times 10^{-6} + 2 \times 10^{-5}$ usw. mol/l) eine dosisabhängige Zunahme der Kontraktionskraft herbeigeführt werden, wobei nach jeder Einzeldosis der Testsubstanz eine konstante Inotropie-Antwort abgewartet wird, bevor die nächsthöhere Konzentration appliziert wird. Die jeweilige Zunahme der Kontraktionskraft wird in % zum Ausgangswert vor der Substanzapplikation ausgedrückt. Als Mass für die kardiotonische Aktivität dient die Konzentration der Testsubstanz, welche die Kontraktionskraft des Vorhofes um 40% über den Ausgangswert verstärkt $[EC_{40pot.}$mol/l], ausgedrückt durch den negativen Logarithmus der $[EC_{40pot.}$mol/l]: –lg$[EC_{40pot.}]$.

Die Toxizitätsuntersuchungen werden an weiblichen NMRI-Mäusen (Körpergewicht 23–30 g) durchgeführt. Die Tiere (5 Tiere pro Dosis) erhalten Futter und Wasser ad libitum. Verschiedene Dosen der Substanzen werden einmal als Suspension in Methocel per Schlundsonde verabreicht. Die Beobachtungsdauer beträgt 7 Tage. Die Dosis tolerata (DT), d.h. die höchste Dosis, bei der noch keine Tiere sterben, wird durch Beobachtung ermittelt. Die mittlere Dosis letalis ($LD_{50}$), d.h. die Dosis, bei der 50% der Tiere sterben, wird aus der Dosis-Wirkungskurve mittels linearer Regression ermittelt.

Die in-vitro-Befunde werden durch die Ergebnisse von in-vivo-Untersuchungen unterstützt. wie aus Messungen der Bronchospasmolyse am Meerschweinchen und der Herzkontraktilität am Meerschweinchen und an der Katze hervorgeht.

Tabelle 11 gibt Ergebnisse der Prüfung der Hemmung des Histamin-induzierten Bronchospasmus am narkotisierten Meerschweinchen wieder. Die Verbindungen 2, 11, 15 und 16 erweisen sich als deutlich wirksamer als Theophyllin.

*Tabelle II:*
Prozentuale Hemmung des Histamin-induzierten Bronchospasmus

| Minuten post applicationem | | 2 | 10 | 20 | 30 | 45 | 60 |
|---|---|---|---|---|---|---|---|
| lfd. Nr. 2 | $Vmax_i$ | 126 | 17 | 9 | 5 | – | – |
| 5 µmol/kg i.v. | $Vmax_e$ | 104 | 16 | 12 | 7 | – | – |
| | $ZV_i$ | 95 | 14 | 8 | 4 | – | – |
| lfd. Nr. 2 | $Vmax_i$ | 143 | 76 | 57 | 37 | – | 17 |
| 20 µmol/kg i.v. | $Vmax_e$ | 118 | 66 | 54 | 44 | – | 23 |
| | $ZV_i$ | 110 | 57 | 43 | 32 | – | 11 |
| lfd. Nr. 2 | $Vmax_i$ | 3 | 47 | 21 | 35 | 40 | 56 |
| 5 µmol/kg i.j. | $Vmax_e$ | 6 | 48 | 30 | 38 | 52 | 60 |
| | $ZV_i$ | 4 | 35 | 27 | 32 | 28 | 57 |
| lfd. Nr. 11 | $Vmax_i$ | 152 | 113 | 89 | 47 | – | 9 |
| 20 µmol/kg i.v. | $Vmax_e$ | 123 | 109 | 63 | 43 | – | 6 |
| | $ZV_i$ | 117 | 84 | 54 | 38 | – | 5 |
| lfd. Nr. 15 | $Vmax_i$ | 172 | 119 | 110 | 82 | – | 60 |
| 20 µmol/kg i.v. | $Vmax_e$ | 108 | 123 | 103 | 84 | – | 46 |
| | $ZV_i$ | 99 | 107 | 104 | 63 | – | 33 |

*Tabelle II:*
Prozentuale Hemmung des Histamin-induzierten Bronchospasmus

| | | | | | | | |
|---|---|---|---|---|---|---|---|
| lfd. Nr. 16 | $Vmax_i$ | 154 | 73 | 48 | 34 | – | 15 |
| 20 µmol/kg i.v. | $Vmax_e$ | 145 | 63 | 60 | 33 | – | 16 |
| | $ZV_i$ | 153 | 66 | 46 | 28 | – | 13 |
| Theophyllin | $Vmax_i$ | 49 | 11 | 8 | 8 | – | – |
| 20 µmol/kg i.v. | $Vmax_e$ | 51 | 10 | 9 | 13 | – | – |
| | $ZV_i$ | 45 | 5 | 5 | 10 | – | – |
| Theophyllin | $Vmax_i$ | 95 | 38 | 19 | 18 | – | 7 |
| 60 µmol/kg i.v. | $Vmax_e$ | 79 | 27 | 15 | 15 | – | 8 |
| | $ZV_i$ | 78 | 27 | 16 | 16 | – | 3 |
| Theophyllin | $Vmax_i$ | 6 | 42 | 46 | 45 | 39 | 39 |
| 60 µmol /kg i.j. | $Vmax_e$ | 8 | 33 | 41 | 40 | 33 | 32 |
| | $ZV_i$ | 7 | 35 | 41 | 37 | 33 | 37 |

$Vmax_i$... maximale Strömungsgeschwindigkeit der Atemluft während der Inspiration
$Vmax_e$... maximale Strömungsgeschwindigkeit der Atemluft während der Exspiration
$ZV_i$... Atemzugvolumen

Es wurde eine Methode zur simultanen Registrierung von pharmakodynamischen bzw. toxischen Effekten an inneren sensiblen Rezeptoren, auf die Atmung und am Herz-Kreislauf-System vom Meerschweinchen verwendet [U. Kilian, E.Müller, E.Ch.Dittmann und J.Hamacher, Arzneimittel-Forschung 28 (II) 1699–1708, 1978]. An narkotisierten (Ethylurethan 1,25 g/kg i.p.), monovagotomierten, spontanatmenden Meerschweinchen (♂, 350–450 g) wurde das Pneumotachogramm registriert. Zur Charakterisierung des durch Histamin (0,09–0,18 µ mol/kg i.v.) ausgelösten Bronchospasmus wurden die maximale Strömungsgeschwindigkeit der Atemluft während Inspiration ($Vmax_i$) und Exspiration ($Vmax_e$) und das Atemzugvolumen ($ZV_i$) gemessen. Ein Histaminspasmus vor Substanzgabe wurde mit mehreren Histaminspasmen nach Substanzgabe verglichen. Die Prüfsubstanzen wurden intravenös (i.v) und/oder intrajejunal (i.j.) appliziert. Pro Messung wurden 5 bis 10 Tiere eingesetzt, aus den Ergebnissen wurde der arithmetische Mittelwert errechnet.

*Tabelle III:*
Schutzwirkung gegen den Acetylcholin-induzierten Bronchospasmus am wachen Meerschweinchen

| lfd. Nr. | Dosis [µmol /kg]p.o. | Versuch 30 Min. p.o. Verdoppelung (Verdreifachung) der Latenzzeit bei N von 10 Tieren |
|---|---|---|
| 1 | 100 | 8 (8) |
| 2 | 60*) | 7 (5)**) |
| 10 | 100 | 6 (6) |
| 10 | 300 | 10 (8) |
| 13 | 100 | 6 (6) |
| 15 | 100 | 10 (10) |
| 16 | 100 | 8 (5) |
| Theophyllin | 100 | 6 (4)***) |

*) appliziert in 4%iger Methocel-Suspension
**) Versuch 45 Min. p.a.: 5(5)
***)Versuch 45 Min. p.a.: 3(2)

In Klammern ist angegeben, bei wie vielen von 10 Tieren sich die Latenzzeit mehr als verdreifacht.

Unter Latenzzeit wird die Zeit von Beginn der Acetylcholin-Vernebelung bis zum Auftreten deutlicher Asthmasymptome verstanden.

Aus der Tabelle III ergibt sich, dass die Verbindungen 1, 2, 10, 13, 15 und 16 eine höhere Schutzwirkung gegen den durch Acetylcholin-Vernebelung erzeugten Bronchospasmus beim wachen Meerschweinchen bewirken als die Vergleichssubstanz Theophyllin.

Die Versuchsdurchführung erfolgt in Anlehnung an T. Olsson, Acta Allergologica 26, 438–447(1971): Merrschweinschen (250–350 g) werden in einem verschlossenen Plexiglaszylinder (Volumen 5 l) zweimal im Abstand von 20 Minuten einem Acetylcholin-Nebel (0,06% in 0,9% Natriumchloridlösung; Ultraschallvernebler Heyer Use 77) ausgesetzt. Die Zeit vom Beginn der Vernebelung bis zum Einsetzen deutlicher Atemanstrengungen (unter Umständen hypoxischer Krampfanfall in Seitenlage) wird gemessen und als Latenzzeit bezeichnet.

Im Kontrollversuch (ohne Substanzapplikation) liegt die Latenzzeit bei 2 Minuten. Die Applikation der Prüfsubstanz erfolgt per oral mittels Schlundsonde (Standarddosis 100 µmol/kg, Volumen 1 ml 4%ige Methocelsuspension in 0,9%iger Natriumchloridlösung/kg). Nach 30 Minuten werden die Tiere erneut dem Acetylcholin-Nebel ausgesetzt und die Latenzzeit gemessen. Eine Verlängerung der Latenzzeit auf mindestens die zweifache Länge wird als Schutzwirkung angesehen.

Die nachfolgende Tabelle IV gibt die am Meerschweinchen-Herzen nach Langendorff erhaltenen Ergebnisse mit erfindungsgemässen Substanzen im Vergleich zu Theophyllin, Amrinone und 6-(4-Methoxyphenyl)-3-[2H]pyridazinon (lfd. Nr. 19) wieder.

*Tabelle IV:*
Prozentuale Zunahme des linksventrikulären Druckes und der Herzfrequenz am isoliert-perfundierten Meerschweinchen-Herzen nach Langendorff

| lfd. Nr. | Linksventrikulärer Druck $EC_{20}$[nMol] | $E_{max}$ | nach nMol | Herzfrequenz bei $EC_{20}$ | bei $E_{max}$ |
|---|---|---|---|---|---|
| 2 | 25 | 59 | 300 | 8 | 13 |
| 4 | 22 | 58 | 300 | 6 | 27 |
| 5 | 14 | 85 | 1000 | 3 | 9 |
| 7 | 13 | 86 | 1000 | 3 | 23 |
| 9 | 50 | 53 | 500 | 7 | 21 |
| 10 | 40 | 61 | 500 | 9 | 19 |
| 19 | 160 | 45 | 1000 | 6 | 11 |
| Theophyllin | –*) | 16 | 3000 | – | 5 |
| Amrinone | 1410 | 35 | 3000 | 7 | 8 |

*) $EC_{20}$ wird nicht erreicht

$EC_{20}$: Dosis, die zu einer 20%igen Zunahme des linksventrikulären Druckes führt

$E_{max}$: Maximal erreichbare prozentuale Zunahme des linksventrikulären Druckes bei der angegebenen Dosis der Prüfsubstanz

Die erfindungsgemässen Verbindungen zeigen eine erheblich stärkere linksventrikulär drucksteigernde Wirkung als die Vergleichssubstanzen. Von besonderer Bedeutung ist hierbei, dass die Herzfrequenz nur unerheblich erhöht wird.

Die Messung vom linksventrikulärem Druck, Herzfrequenz und Koronarfluss erfolgt am isoliert-perfundierten Herzen (Langendorff-Herz) von Meerschweinchen (♂ und ♀, 400–500 g) Nach Tötung der Tiere durch Genickschlag und Ausblutung durch die Carotiden wird der Brustraum eröffnet, die Aorta freigelegt und mit einem Faden angeschlungen. Eine mit einem Schlauch an die Apparatur angeschlossene Kanüle, aus der langsam Nährlösung tropft, wird herzwärts eingeführt und das Herz schnell nach der Entnahme aus dem Thorax an die Perfusionsapparatur angeschlossen. Eine Ballonsonde, mit Anschluss an einen Statham-Druckaufnehmer, wird durch einen Schnitt durch den linken Vorhof bis in den linken Ventrikel vorgeschoben und mit einem Vordruck von 40 mm Hg versehen. Die koronare Perfusion erfolgt druckkonstant (60 cm Wassersäule) aus einer Mariott'schen Flasche mit Krebs-Henseleit Lösung (37 °C, Carbogen-Begasung). Die aus dem Koronarsinus in den rechten Vorhof frei abfliessende Nährlösung wird mit einem Flussmesser (Grefe, Lüdenscheid) erfasst und zusammen mit der Änderung des linksventrikulären Druckes und der daraus ermittelten Herzfrequenz auf einem Watanabe-Schreiber registriert Die Substanzen werden in einem Volumen von 0,1 ml Nährlösung in der in Tab. IV angegebenen Dosierung innerhalb von 2 sec. herznah in den Perfusionsschlauch sukzessiv in ansteigender Dosis an 5 Herzen pro Substanz und Dosis appliziert.

Die nachfolgende Tabelle V gibt die Ergebnisse der Messung der maximalen Druckanstiegsgeschwindigkeit im rechten Ventrikel von Meerschweinchen wieder.

*Tabelle V:*

Prozentuale Änderung der Herzfunktion $dP/dt_{max}$ beim Meerschweinchen

| lfd. Nr. | Dosis [µmol/kg]i.v. | $dP/dt_{max}$ [Minuten]post applicationem 0–10 (Maximum) | 10 | 30 |
|---|---|---|---|---|
| 2 | 0,3 | 36 | 5 | 2 |
| | 1 | 115 | 25 | 30 |
| | 3 | 168 | 68 | 47 |
| 4 | 0,3 | 39 | 12 | 23 |
| | 1 | 140 | 26 | 56 |
| | 3 | 195 | 66 | 59 |
| 10 | 0,3 | 42 | 16 | 21 |
| | 1 | 97 | 45 | 46 |
| | 3 | 164 | 90 | 66 |
| Amrinone | 0,3 | 33 | 8 | 13 |
| | 1 | 40 | 21 | 15 |
| | 3 | 66 | 13 | 20 |
| | 10 | 101 | 39 | 29 |
| | 30 | 141 | 78 | 69 |
| Theophyllin | 10 | 59 | 12 | 20 |
| | 30 | 92 | 53 | 67 |
| | 100 | 147 | 135 | 112 |

$dP/dt_{max}$: Maximale Druckanstiegsgeschwindigkeit im rechten Ventrikel

Aus den Werten der Tabelle V ergibt sich, dass die untersuchten erfindungsgemässen Verbindungen stärker positiv inotrop (Zunahme von $dP/dt_{max}$) wirken als die Vergleichssubstanzen Amrinone und Theophyllin.

Die Wirkung der Substanzen auf die Kontraktilität des Herzens von Meerschweinchen wird an Tieren (♂ 400 bis 1000 g) in Urethan-Narkose geprüft. Der Druckablauf im rechten Herzventrikel wird mit einem durch die V. jugularis dextra eingeführten Tip-Katheter gemessen und daraus die maximale Druckanstiegsgeschwindigkeit ($dP/dt_{max}$) ermittelt. Die Herzfrequenz wird von den Druckwellen abgeleitet. Es wurden keine auffälligen Erhöhungen der Herzfrequenz festgestellt. Die Prüfsubstanzen wurden intravenös appliziert. Pro Messung wurden 5–7 Tiere eingesetzt, und aus den Ergebnissen wurde der arithmetische Mittelwert errechnet.

Die nachfolgende Tabelle VI gibt Ergebnisse der Messung der prozentualen maximalen Druckanstiegsgeschwindigkeit am linken Ventrikel an Katzen wieder. Als Vergleichsverbindungen wurden Amrinone und Theophyllin-Ethylendiamin untersucht.

*Tabelle VI*

Prozentuale Änderung der Herzfunktion $dP/dt_{max}$ bei der Katze

| lfd. Nr. | Dosis [µmol/kg] i.V. | $dP/dt_{max}$ 3 | 10 | 30 | 60 [Minuten post applicationem] |
|---|---|---|---|---|---|
| 2 | 0,3 | 55 | 31 | 26 | 37 |
| | 1 | 89 | 57 | 30 | 31 |
| 5 | 0,3 | 27 | 12 | 11 | 13 |
| | 1 | 64 | 25 | 15 | 20 |
| 7 | 0,3 | 36 | 4 | 1 | 2 |
| | 1 | 90 | 25 | 11 | 11 |
| Theophyllin-Ethylendiamin | 3 | 8 | 2 | 2 | 1 |
| | 10 | 62 | 30 | 22 | 26 |
| Amrinone | 1 | 17 | 6 | 7 | 23 |
| | 3 | 52 | 42 | 40 | 43 |
| | 10 | 70 | 67 | 59 | 55 |

$dP/dt_{max}$: Maximale Druckanstiegsgeschwindigkeit im linken Ventrikel

Die Dosisangabe bei Theophyllin-Ethylendiamin bezieht sich auf den Theophyllin-Gehalt.

Die Substanzen 2, 5 und 7 wirken stärker positiv inotrop (Zunahme von $dP/dt_{max}$) als die Vergleichsverbindungen, ohne zu einer wesentlichen Zunahme von Herzfrequenz und enddiastolischem Ventrikeldruck zu führen.

Die Wirkung der Substanzen auf die Kontraktilität des Herzens an Katzen wurde an Tieren (♂, ♀, 3, 2 bis 5,2 kg) in Chloralose/Urethan-Narkose geprüft. Der Druckablauf im linken Herzventrikel wurde mit einem durch die A. carotis dextra eingeführten Tip-Katheter gemessen, wobei die R-Zacke des EKG's (bipolare Brustwandableitung) ein Signal zur Registrierung des enddiastolischen Drucks auslöst; zudem wurde aus dem Druckablauf die maximale Druckanstiegsgeschwindigkeit ($dP/dt_{max}$) ermittelt. Die Herzfrequenz wurde von den Druckwellen abgeleitet. Die Prüfsubstanzen wurden intravenös appliziert. Pro Messung wurden 2–6 Tiere eingesetzt, aus den Ergebnissen wurde der arithmetische Mittelwert errechnet.

Messungen von Blutdruck und Herzfrequenz nach Gabe erfindungsgemässer Verbindungen an narkotisierten Ratten ergaben keine nachteiligen Befunde.

**Patentansprüche für die Vertragsstaaten: BE, CH, DE, FR, GB, IT, LI, LU, NL, SE**

1. 6-Aryl-3-[2H]pyridazinone der allgemeinen Formel I

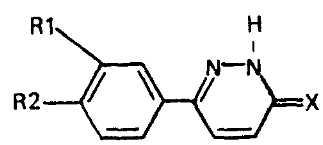

(I),

worin einer der Substituenten R1 oder R2 eine Methoxygruppe und der andere eine Alkoxygruppe mit 2 bis 5 Kohlenstoffatomen oder eine Alkenyloxygruppe mit 3 bis 5 Kohlenstoffatomen und X ein Sauerstoffatom oder ein Schwefelatom bedeuten, und ihre pharmakologisch verträglichen Salze mit Basen.

2. 6-Aryl-3[2H]pyridazinone der allgemeinen Formel Ia

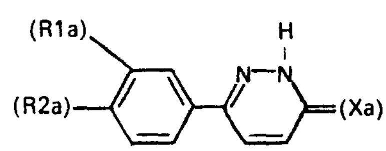

(Ia),

worin

R1a eine Methoxygruppe,

R2a eine Alkoxygruppe mit 2 bis 4 Kohlenstoffatomen oder eine Alkenyloxygruppe mit 3 oder 4 Kohlenstoffatomen und

Xa ein Sauerstoffatom bedeuten,

und ihre pharmakologisch verträglichen Salze mit Basen.

3. 6-Aryl-3[2H]pyridazinone der allgemeinen Formel Ib

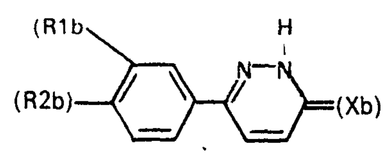

(Ib),

worin
R1b eine Alkoxygruppe mit 2 bis 4 Kohlenstoffatomen oder eine Alkenyloxygruppe mit 3 oder 4 Kohlenstoffatomen,
R2b eine Methoxygruppe und
Xb ein Sauerstoffatom bedeuten,
und ihre pharmakologisch verträglichen Salze mit Basen.

4. Verbindungen nach Anspruch 1, in denen X ein Sauerstoffatom bedeutet.

5. 6-(3-Methoxy-4-n-propoxyphenyl)-3[2H]pyridazinon.

6. 6-(3-Isobutoxy-4-methoxyphenyl)-3[2H]-pyridazinon.

7. Verfahren zur Herstellung von 6-Aryl-3-[2H]pyridazinonen der allgemeinen Formel I und ihren pharmakologisch verträglichen Salzen mit Basen, dadurch gekennzeichnet, dass man

a) ein 6-Aryl-tetrahydropyridazinon der allgemeinen Formel II

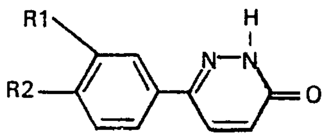 (II),

worin R1 und R2 die in Anspruch 1 angegebene Bedeutung haben, oxydiert, oder

b) eine Morpholinobuttersäure der allgemeinen Formel III

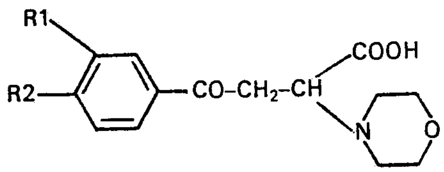 (III),

worin R1 und R2 die in Anspruch 1 angegebene Bedeutung haben, mit Hydrazin umsetzt, oder

c) eine Acrylsäure der allgemeinen Formel IV

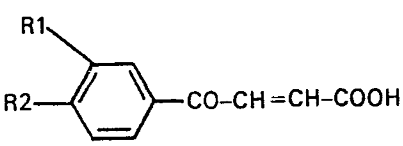 (IV),

worin R1 und R2 die in Anspruch 1 angegebene Bedeutung haben, mit Hydrazin umsetzt.

und jeweils gewünschtenfalls anschliessend das erhaltene Pyridazinon I (X=O) in das Pyridazinthion I (X=S) und/oder in das Salz überführt.

8. Verfahren zur Herstellung der Verbindungen des Anspruchs 2, dadurch gekennzeichnet, dass man

a) ein Aryl-tetrahydropyridazinon der Formel IIa

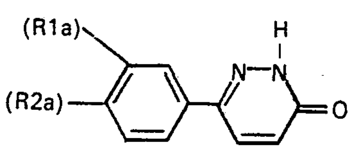 (IIa),

worin R1a und R2a die in Anspruch 2 angegebene Bedeutung haben, oxydiert, oder

b) eine Morpholinobuttersäure der Formel IIIa

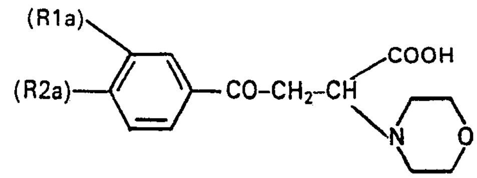 (IIIa),

worin R1a und R2a die in Anspruch 2 angegebene Bedeutung haben, mit Hydrazin umsetzt oder

c) eine Acrylsäure der Formel IVa

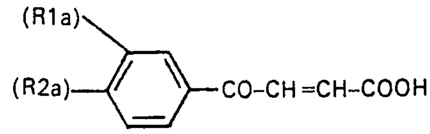 (IVa),

worin R1a und R2a die in Anspruch 2 angegebene Bedeutung haben, mit Hydrazin umsetzt,

und jeweils gewünschtenfalls anschliessend das erhaltene Pyridazinon Ia in das Salz überführt.

9. Verfahren zur Herstellung der Verbindungen des Anspruches 3, dadurch gekennzeichnet, dass man

a) ein Aryl-tetrahydropyridazinon der Formel IIb

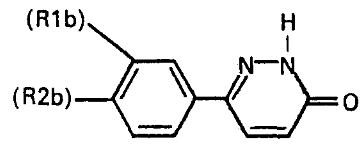 (IIb),

worin R1b und R2b die in Anspruch 3 angegebene Bedeutung haben, oxydiert, oder

b) eine Morpholinobuttersäure der Formel IIIb

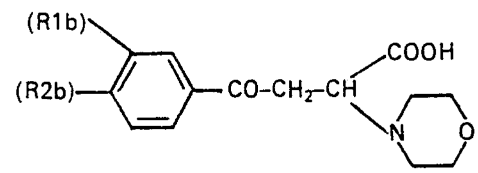 (IIIb),

worin R1b und R2b die in Anspruch 3 angegebene Bedeutung haben, mit Hydrazin umsetzt,

c) eine Acrylsäure der Formel IVb

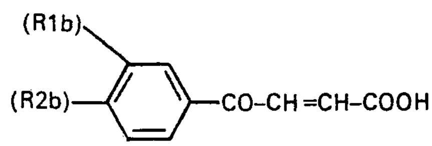 (IVb),

worin R1b und R2b die in Anspruch 3 angegebene Bedeutung haben, mit Hydrazin umsetzt,

und jeweils gewünschtenfalls anschliessend das so erhaltene Pyridazinon Ib in das Salz überführt.

10. Arzneimittel, enthaltend eine oder mehrere Verbindungen gemäss Anspruch 1.

11. Verwendung von Verbindungen gemäss Anspruch 1 bei der Herstellung von Arzneimitteln zur Behandlung oder Prophylaxe von Krankheiten, die auf Erkrankungen der Bronchien beruhen.

12. Verwendung von 6-(3-Methoxy-4-n-propoxyphenyl)-3[2H]pyridazinon bei der Herstellung von Arzneimitteln zur Behandlung oder Prophylaxe von Krankheiten, die auf Erkrankungen der Bronchien beruhen.

13. Verwendung von Verbindungen gemäss Anspruch 1 bei der Herstellung von Arzneimitteln zur Behandlung von Krankheiten, die auf einer Insuffizienz des Herzens beruhen, oder zur Stärkung des Herzens.

14. Verwendung von 6-(3-Isobutoxy-4-methoxyphenyl)-3[2H]pyridazinon bei der Herstellung von Arzneimitteln zur Behandlung von Krankheiten, die auf einer Insuffizienz des Herzens beruhen, oder zur Stärkung des Herzens.

**Patentansprüche für den Vertragsstaat AT**

1. Verfahren zur Herstellung von 6-Aryl-3[2H]pyridazinonen der allgemeinen Formel I

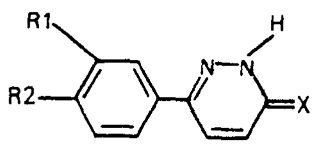

(I),

worin einer der Substituenten R1 oder R2 eine Methoxygruppe und der andere eine Alkoxygruppe mit 2 bis 5 Kohlenstoffatomen oder eine Alkenyloxygruppe mit 3 bis 5 Kohlenstoffatomen und X ein Sauerstoffatom oder ein Schwefelatom bedeuten, und ihren pharmakologisch verträglichen Salzen mit Basen, dadurch gekennzeichnet, dass man

a) ein 6-Aryl-tetrahydropyridazinon der allgemeinen Formel II

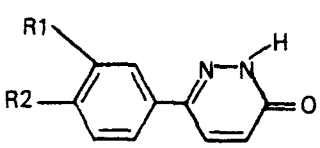

(II),

worin R1 und R2 die oben angegebene Bedeutung haben, oxydiert, oder

b) eine Morpholinobuttersäure der allgemeinen Formel III

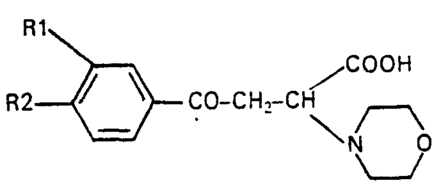

(III),

worin R1 und R2 die oben angegebene Bedeutung haben, mit Hydrazin umsetzt, oder

c) eine Acrylsäure der allgemeinen Formel IV

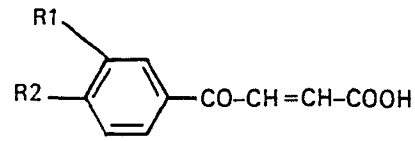

(IV),

worin R1 und R2 die oben angegebene Bedeutung haben, mit Hydrazin umsetzt,

und jeweils gewünschtenfalls anschliessend das erhaltene Pyridazinon I (X=O) in das Pyridazinthion I (X=S) und/oder in das Salz überführt.

2. Verfahren zur Herstellung von 6-Aryl-3[2H]pyridazinonen der allgemeinen Formel Ia

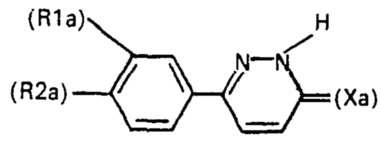

(Ia),

worin

R1a eine Methoxygruppe,

R2a eine Alkoxygruppe mit 2 bis 4 Kohlenstoffatomen oder eine Alkenyloxygruppe mit 3 oder 4 Kohlenstoffatomen und

Xa ein Sauerstoffatom bedeuten,

und ihren pharmakologisch verträglichen Salzen mit Basen, dadurch gekennzeichnet, dass man

a) ein Aryl-tetrahydropyridazinon der allgemeinen Formel IIa

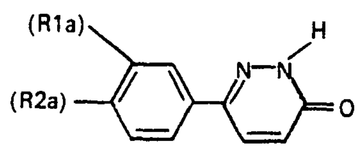

(IIa),

worin R1a und R2a die oben angegebene Bedeutung haben, oxydiert, oder

b) eine Morpholinobuttersäure der allgemeinen Formel IIIa

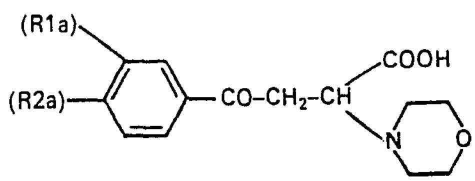

(IIIa),

worin R1a und R2a die oben angegebene Bedeutung haben, mit Hydrazin umsetzt, oder

c) eine Acrylsäure der allgemeinen Formel IVa

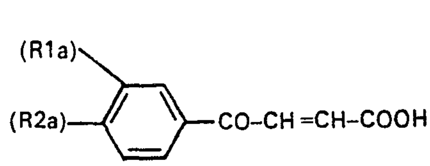

(IVa),

worin R1a und R2a die oben angegebene Bedeutung haben, mit Hydrazin umsetzt,

und jeweils gewünschtenfalls anschliessend das erhaltene Pyridazinon Ia in das Salz überführt.

3. Verfahren zur Herstellung von 6-Aryl-3-[2H]pyridazinonen der allgemeinen Formel Ib

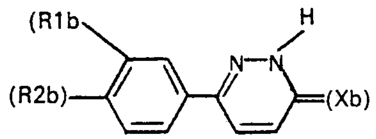

(Ib),

worin

R1b eine Alkoxygruppe mit 2 bis 4 Kohlenstoffatomen oder eine Alkenyloxygruppe mit 3 oder 4 Kohlenstoffatomen,

R2b eine Methoxygruppe und

Xb ein Sauerstoffatom bedeuten,

und ihren pharmakologisch verträglichen Salzen mit Basen, dadurch gekennzeichnet, dass man

a) ein Acryl-tetrahydropyridazinon der allgemeinen Formel IIb

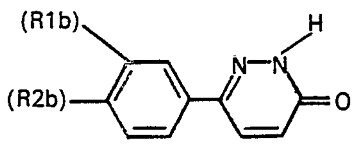

(IIb),

worin R1b und R2b die oben angegebene Bedeutung haben, oxydiert oder

b) eine Morpholinobuttersäure der allgemeinen Formel IIIb

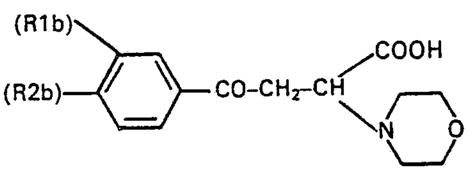

(IIIb),

worin R1b und R2b die oben angegebene Bedeutung haben, mit Hydrazin umsetzt, oder

c) eine Acrylsäure der allgemeinen Formel IVb

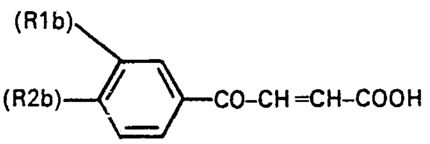

(IVb),

worin R1b und R2b die oben angegebene Bedeutung haben, mit Hydrazin umsetzt,

und jeweils gewünschtenfalls anschliessend das so erhaltene Pyridazinon Ib in das Salz überführt.

4. Verfahren zur Herstellung von 6-(3-Methoxy-4-n-propoxyphenyl)-3[2H]pyridazinon und dessen pharmakologisch verträglichen Salzen mit Basen, dadurch gekennzeichnet, dass man

a) 6-(3-Methoxy-4-n-propoxyphenyl)-4,5-dihydro-3[2H]pyridazinon oxydiert, oder

b) 4-(3-Methoxy-4-n-propoxyphenyl)-2-morpholino-4-oxobuttersäure mit Hydrazin umsetzt, oder

c) 3-(3-Methoxy-4-n-propoxybenzoyl)-acrylsäure mit Hydrazin umsetzt, und gewünschtenfalls anschliessend das erhaltene Pyridazinon in das Salz überführt.

5. Verfahren zur Herstellung von 6-(3-Isobutoxy-4-methoxyphenyl)-3[2H]pyridazinon und dessen pharmakologisch verträglichen Salzen mit Basen, dadurch gekennzeichnet, dass man

a) 6-(3-Isobutoxy-4-methoxyphenyl)-4,5-dihydro-3[2H]pyridazinon oxydiert, oder

b) 4-(3-Isobutoxy-4-methoxyphenyl)-2-morpholino-4-oxobuttersäure mit Hydrazin umsetzt, oder

c) 3-(3-Isobutoxy-4-methoxybenzoyl)-acrylsäure mit Hydrazin umsetzt, und gewünschtenfalls anschliessend das erhaltene Pyridazinon in das Salz überführt.

6. Verfahren zur Herstellung von Arzneimitteln zur Behandlung und Prophylaxe von Krankheiten, die auf Erkrankungen der Bronchien beruhen, dadurch gekennzeichnet, dass man ein oder mehrere 6-Aryl-3[2H]pyridazinone I, Ia, Ib und/oder deren pharmakologisch verträglichen Salze mit Basen durch Mischen mit geeigneten pharmazeutisch annehmbaren Trägerstoffen in eine zur Verabreichung als Arzneimittel geeignete Form bringt.

7. Verwendung der 6-Aryl-3[2H]pyridazinone der in Anspruch 1 angegebenen Formel I, worin R1 und R2 die in Anspruch 1 angegebenen Bedeutungen haben und deren pharmakologisch verträglichen Salze mit Basen bei der Herstellung von Arzneimitteln zur Behandlung oder Prophylaxe von Krankheiten, die auf Erkrankungen der Bronchien beruhen.

8. Verwendung von 6-(3-Methoxy-4-n-propoxyphenyl)-3 [2H]pyridazinon und 6-(3-Isobutoxy-4-methoxyphenyl)-3[2H]pyridazinon und deren Salze mit Basen bei der Herstellung von Arzneimitteln zur Behandlung von Krankheiten, die auf Erkrankungen der Bronchien beruhen.

9. Verwendung der 6-Aryl-3[2H]pyridazinone der in Anspruch 1 angegebenen Formel I, worin R1 und R2 die in Anspruch 1 angegebenen Bedeutungen haben und deren pharmakologisch verträglichen Salze mit Basen bei der Herstellung von Arzneimitteln zur Behandlung von Krankheiten, die auf Insuffizienz des Herzens beruhen, oder zur Stärkung des Herzens.

10. Verwendung von 6 - (3-Isobutoxy- 4 - methoxyphenyl)-3[2H]pyridazinon und dessen Salze mit Basen bei der Herstellung von Arzneimitteln zur Behandlung von Krankheiten, die auf Insuffizienz des Herzens beruhen, oder zur Stärkung des Herzens.

**Revendications pour les Etats contractants: BE, CH, DE, FR, GB, IT, LI, LU, NL, SE**

1. 6-Aryl-3[2H]pyridazinones de formule générale I

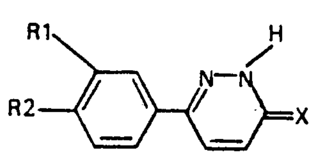

(I),

dans laquelle l'un des substituants R1 ou R2 représente un groupe méthoxy, tandis que l'autre représente un groupe alcoxy ayant 2 à 5 atomes de carbone ou un groupe alcényloxy ayant 3 à 5 atomes de carbone et X représente un atome d'oxygène ou un atome de soufre, et leurs sels, pharmacologiquement compatibels, avec des bases.

2. 6-Aryl-3[2H]pyridazinones de formule générale Ia

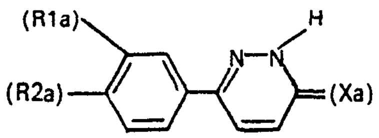

(Ia),

dans laquelle

R1a représente un groupe méthoxy,

R2a représente une groupe alcoxy ayant 2 à 4 atomes de carbone ou un groupe alcényloxy ayant 3 ou 4 atomes de carbone et

Xa représente un atome d'oxygène,

et leurs sels, pharmacologiquement compatibles, avec des bases.

3. 6-Aryl-3[2H]pyridazinones de formule générale Ib

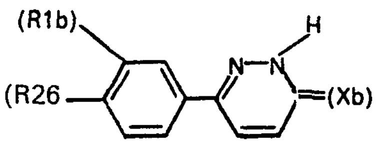

(Ib),

dans laquelle

R1b représente un groupe alcoxy ayant 2 à 4 atomes de carbone ou un groupe alcényloxy ayant 3 ou 4 atomes de carbone,

R2b représente un groupe méthoxy et

Xb représente un atome d'oxygène,

et leurs sels, pharmacologiquement compatibles, avec des bases.

4. Composés selon la Revendication 1, dans lesquelles X représente un atome d'oxygène.

5. 6-(3-Méthoxy-4-n-propoxyphényl)-3[2]-pyridazinone.

6. 6-(3-Isobutoxy-4-méthoxyphényl) -3[2H]-pyridazinone.

7. Procédé pour la préparation de 6-aryl-3[2H]pyridazinones de formule générale I et leurs sels, pharmacologiquement compatibles, avec des bases, caractérisé en ce que

a) on soumet à une oxydation une 6-aryl-tétrahydropyridazinone de formule générale II

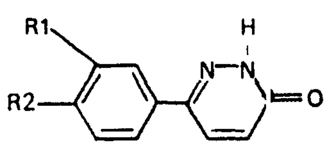

(II),

dans laquelle R1 et R2 sont définis comme spécifié dans la Revendication 1, ou

b) on fait réagir un acide morpholinobutyrique de formule générale III

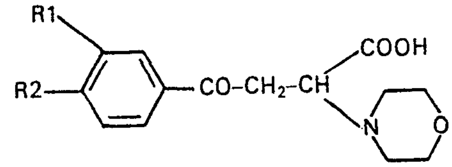

(III),

dans laquelle R1 et R2 sont définis comme spécifié ci-dessus, avec l'hydrazine, ou

c) on fait réagir un acide acrylique de formule générale IV

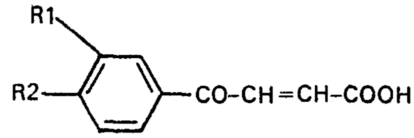

(IV),

dans laquelle R1 et R2 sont définis comme spécifié dans la Revendication 1, avec l'hydrazine,

et, si on le désire, on transforme consécutivement de manière respective la pyridazinone I (X=O) obtenue en pyridazinethione I (X=S) et/ou en son sel.

8. Procédé pour la préparation des composés de la Revendication 2, caractérisé en ce que

a) on soumet à une oxydation une aryl-tétrahydropyridazinone de formule générale IIa

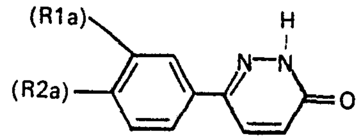

(IIa),

dans laquelle R1a et R2a sont définis comme spécifié dans la Revendication 2, ou

b) on fait réagir un acide morpholinobutyrique de formule générale IIIa

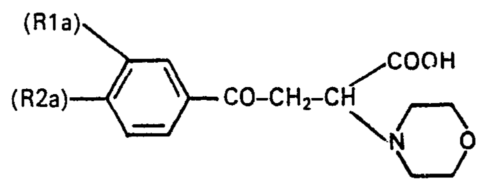

(IIIa),

dans laquelle R1a et R2a sont définis comme spécifié dans la Revendication 2, avec l'hydrazine, ou

c) on fait réagir un acide acrylique de formule générale IVa

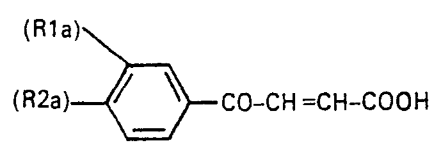

(IVa),

dans laqeulle R1a et R2a sont définis comme spécifié dans la Revendication 2, avec l'hydrazine,

et, si on le désire, on transforme consécutivement de manière respective la pyridazinone Ia obtenue en son sel.

9. Procédé pour la préparation des composés selon la Revendication 3, caractérisé en ce que

a) on soumet à une oxydation une aryl-tétrahydropyridazinone de formule générale IIb

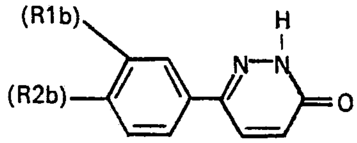

(IIb),

dans laquelle R1b et R2b sont définis comme spécifié dans la Revendication 3, ou

b) on fait réagir un acide morpholinobutyrique de formule générale IIIb

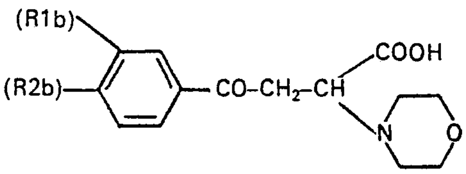

(IIIb),

dans laquelle R1b et R2b sont définis comme spécifié dans la Revendication 3, avec l'hydrazine, ou

c) on fait réagir un acide acrylique de formule générale IVb

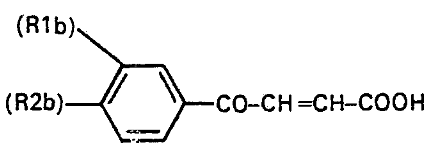

(IVb),

dans laquelle R1b et R2b sont définis comme spécifié dans la Revendication 3, avec l'hydrazine,

et, si on le désire, on transforme consécutivement de manière respective la pyridazinone Ib ainsi obtenue en son sel.

10. Médicaments, contenant un ou plusieurs composés selon la Revendications 1.

11. Application des composés selon la Revendication 1, à la préparation de médicaments pour le traitement ou la prophylaxie de maladies dues aux infections des bronches.

12. Application de la 6-(3-méthoxy-4-n-propoxyphényl)-3[2H]pyridazinone à la préparation de médicaments pour le traitement ou la prophylaxie de maladies dues aux infections des bronches.

13. Application des composés selon la Revendication 1 à la préparation de médicaments pour le traitement de maladies qui sont dues à une insuffisance cardiaque ou destinés à tonifier le cœur.

14. Application de la 6-(3-isobutoxy-4-méthoxyphényl)-3[2H]pyridazinone à la préparation de médicaments pour le traitement de maladies dues à une insuffisance du cœur ou destinés à tonifier le cœur.

**Revendications pour l'Etat contractant: AT**

1. Procédé pour la préparation de 6-aryl-3[2H]pyridazinones de formule générale I

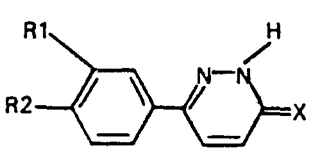

(I),

dans laquelle l'un des substituants R1 ou R2 représente un groupe méthoxy, tandis que l'autre représente un groupe alcoxy ayant 2 à 5 atomes de carbone ou un groupe alcényloxy ayant 3 à 5 atomes de carbone et X représente un atome d'oxygène ou un atome de soufre, et leurs sels, pharmacologiquement compatibles, avec des bases, caractérisé en ce que

a) on soumet à une oxydation une 6-aryl-tétrahydropyridazinone de formule générale II

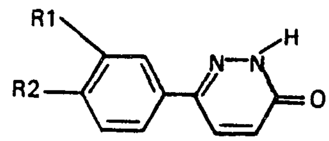

(II),

dans laquelle R1 et R2 sont définis comme spécifié ci-dessus, ou

b) on fait réagir un acide morpholinobutyrique de formule générale III

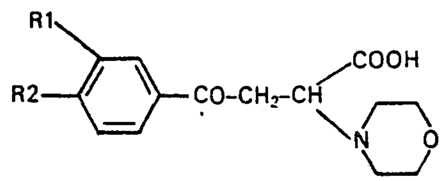

(III),

dans laquelle R1 et R2 sont définis comme spécifié ci-dessus, avec l'hydrazine, ou

c) on fait réagir un acide acrylique de formule générale IV

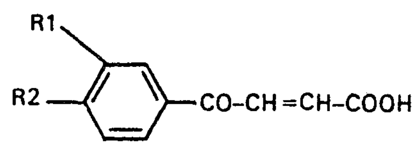

(IV),

dans laquelle R1 et R2 sont définis comme spécifiés ci-dessus, avec l'hydrane, et, si on le désire, on transforme consécutivement de manière respective la pyridazinone 1 (X=O) obtenue en pyridazinethione I (X=S) et/ou en son sel.

2. Procédé pour la préparation de 6-aryl-3[2H]pyridazinones de formule générale Ia

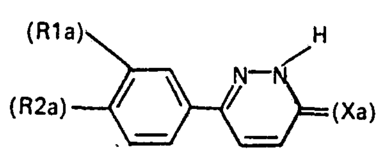

(Ia),

dans laquelle

R1a représente un groupe méthoxy,

R2a représente un groupe alcoxy ayant 2 à 4 atomes de carbone ou un groupe alcényloxy ayant 3 ou 4 atomes de carbone et

Xa représente un atome d'oxygène,

et leurs sels, pharmacologiquement compatibles, avec des bases, caractérisé en ce que

a) on soumet à une oxydation une aryl-tétrahydropyridazinone de formule générale IIa

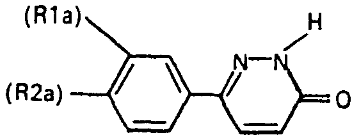

(IIa),

dans laquelle R1a et R2a sont définis comme spécifié ci-dessus, ou

b) on fait réagir un acide morpholinobutyrique de formule générale IIIa

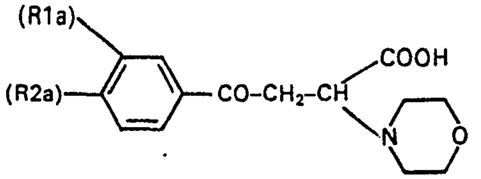

(IIIa),

dans laquelle R1a et R2a sont définis comme spécifié ci-dessus, avec l'hydrazine, ou

c) on fait réagir un acide acrylique de formule générale IVa

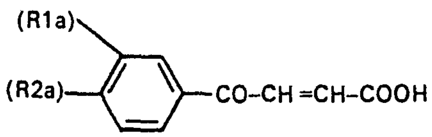

(IVa),

dans laquelle R1a et R2a sont définis comme spécifié ci-dessus, avec l'hydrazine, et, si on le désire, on transforme consécutivement de manière respective la pyridazinone Ia obtenue en son sel.

3. Procédé pour la préparation de 6-aryl-3[2H]pyridazinones de formule générale Ib

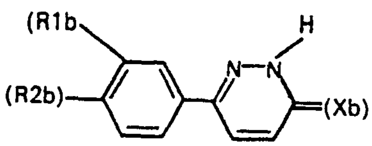

(Ib),

dans laquelle

R1b représente un groupe alcoxy ayant 2 à 4 atomes de carbone ou un groupe alcényloxy ayant 3 ou 4 atomes de carbone,

R2b représente un groupe méthoxy et

Xb représente un atome d'oxygène,

et leurs sels, pharmacologiquement compatibles, avec des bases, caractérisé en ce que

a) on soumet à une oxydation une aryl-tétrahydropyridazinone de formule générale IIb

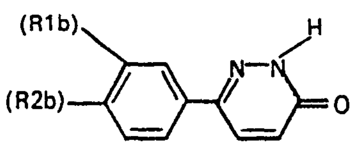

(IIb),

dans laquelle R1b et R2b sont définis comme spécifé ci-dessus, ou

b) on fait réagir un acide morpholinobutyrique de formule générale IIIb

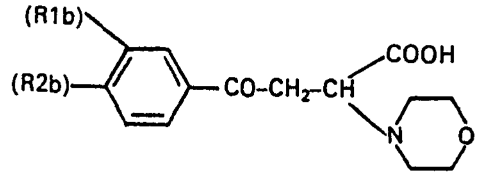

(IIIb),

dans laquelle R1b et R2b sont définis comme spécifié ci-dessus, avec l'hydrazine ou

c) on fait réagir un acide acrylique de formule générale IVb

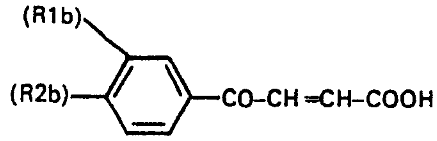

(IVb),

dans laquelle R1b et R2b sont définis comme spécifié ci-dessus, avec l'hydrazine,

et , si on le désire, on transforme consécutivement de manière respective la pyridazinone Ib ainsi obtenue en son sel.

4. Procédé pour la préparation de la 6-(3-méthoxy-4-n-propoxyphényl)-3[2H]pyridazinone et ses sels, pharmacologiquement compatibles avec des bases, caractérisé en ce que

a) on soumet à une oxydation la 6 - (3-méthoxy-4-n-propoxyphényl) - 4,5 - dihydro-3[2H]pyridazinone ou

b) on fait réagir l'acide 4-(3-méthoxy-4-n-propoxyphényl)-2-morpholino-4-oxobutyrique avec l'hydrazine, ou

c) on fait réagir l'acide 3-(3-méthoxy-4-n-propoxybenzoyl)-acrylique avec l'hydrazine,

et, si on le désire, on transforme consécutivement la pyridazinone obtenue en son sel.

5. Procédé pour la préparation de la 6-(3-Isobutoxy-4-méthoxyphényl-3[2H]pyridazinone et ses sels, pharmacologiquement compatibles, avec des bases, caractérisé en ce que

a) on soumet à une oxydation la 6-(3-isobutoxy)-4-méthoxyphényl)-4,5-dihydro-3[2H]pyridazinone, ou

b) on fait réagir l'acide 4-(3-isobutoxy-4-méthoxyphényl)-2-morpholino-4-oxobutyrique avec l'hydrazine, ou

c) on fait réagir l'acide 3-(3-isobutoxy-4-méthoxybenzoyl)-acrylique avec l'hydrazine,

et, si on le désire, on transforme consécutivement la pyridazinone obtenue en son sel.

6. Procédé pour la préparation de médicaments pour le traitement et la prophylaxie de maladies qui sont dues aux affections des bronches, caractérisé en ce que l'on convertit une ou plusieurs 6-aryl-3[2H]pyridazinones I, Ia, Ib et/ou leurs sels, pharmacologiquemen compatibles, avec des bases par mélange avec des véhicules pharmaceutiquement acceptables appropriés, sous une forme convenable pour l'administration en tant que médicaments.

7. Application des 6-aryl-3[2H pyridazinones de formule I indiquée dans la Revendication 1, dans laquelles R1 et R2 sont définis comme spécifié dans la revendication 1 et leurs sels, pharmacologiquement compatibles, avec des bases à la préparation de médicaments pour le traitement ou la prophylaxie de maladies qui sont dues aux affections des bronches.

8. Application de la 6-(3-méthoxy-4-n-propoxyphényl)-3[2H]pyridazinone et de la 6-(3-isobutoxy- 4 -méthoxyphényl) -3[2H]pyridazinone et leurs sels avec des bases, à la préparation de médicaments pour le traitement de maladies qui sont dues aux affections des bronches.

9. Application des 6-aryl-3[2H] pyridazinones de formule I indiquée dans la Revendication 1, dans laquelles R1 et R2 sont définis comme spécifié dans la Revendication 1 et leurs sels, pharmacologiquement compatibles, avec des bases, à la préparation de médicaments pour le traitement de maladies qui sont dues à une insuffisance du cœur, ou qui sont destinés à tonifier le cœur.

10. Application de la 6-(3-isobutoxy-4-méthoxyphényl)-3[2H]pyridazinone et ses sels avec des bases, à la préparation de médicaments pour le traitement de maladies qui sont dues à une insuffisance du cœur, ou qui sont destinés à tonifier le cœur.

**Claims for the contracting states BE, CH, DE, FR, GB, IT, LI, LU, NL, SE**

1. A 6-aryl-3[2H]pyridazinone of the general formula I

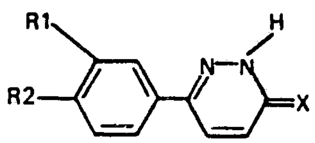

(I),

in which one of the substituents R1 or R2 denotes a methoxy group and the other denotes an alkoxy group with 2 to 5 carbon atoms or an alkenyloxy group with 3 to 5 carbon atoms, and X denotes an oxygen atom or a sulfur atom, or a pharmacologically acceptable salt thereof with a base.

2. A 6-aryl-3[2H]-pyridazinone of the general formula Ia

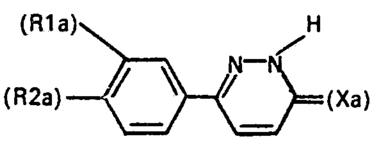

(Ia),

win which R1a denotes a methoxy group, R2a denotes an alkoxy group with 2 to 4 carbon atoms or an alkenyloxy group with 3 or 4 carbon atoms and Xa denotes an oxygen atom, or a pharmacologically acceptable salt thereof with a base.

3. A 6-aryl-3[2H]-pyridazinone of the general formula Ib

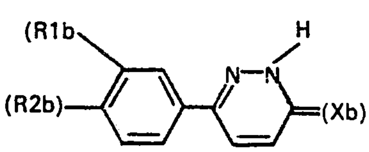

(Ib),

in which R1b denotes an alkoxy group with 2 to 4 carbon atoms or an alkenyloxy group with 3 or 4 carbon atoms, R2b denotes a methoxy group and Xb denotes an oxygen atom, or a pharmacologically acceptable salt thereof with a base.

4. A compound as claimed in claim 1, in which X denotes an oxygen atom.

5. 6-(3-Methoxy-4-n-propoxyphenyl)-3[2H]-pyridazinone.

6. 6-(3-Isobutoxy-4-methoxyphenyl)-3[2H]-pyridazinone.

7. A process for the preparation of a 6-aryl-3[2H]-pyridazinone of the general formula I or a pharmacologically acceptable salt thereof with a base, which comprises

a) oxidizing a 6-aryl-tetrahydropyridazinone of the general formula II

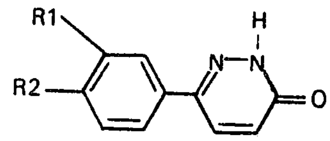

(II),

in which R1 and R2 have the meaning given in claim 1, or b) reacting a morpholinobutyric acid of the general formula III

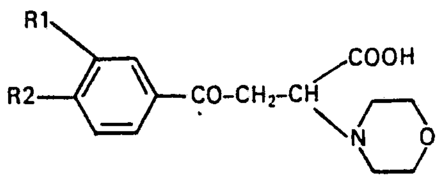

(III),

in which R1 and R2 have the meaning given in claim 1, with hydrazine, or

c) reacting an acrylic acid of the general formula IV

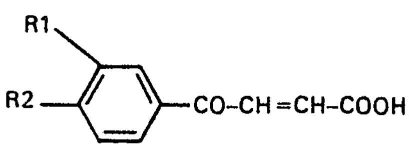

(IV)

in which R1 and R2 have the meaning given in claim 1, with hydrazine, and in each case, if desired, subsequently converting the resulting pyridazinone I (X=O) into the pyridazinethione I (X=S) and/or into the salt.

8. A process for the preparation of a compound of claim 2, which comprises

a) oxidizing an aryl-tetrahydropyridazinone of the formula IIa

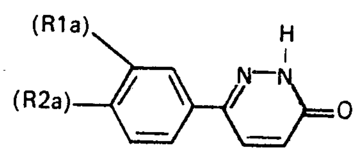

(IIa),

in which R1a und R2a have the meaning given in claim 2, or

b) reacting a morpholinobutyric acid of the formula IIIa

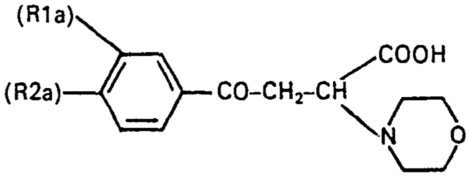 (IIIa),

in which R1a and R2a have the meaning given in claim 2, with hydrazine, or

c) reacting an acrylic acid of the formula IVa

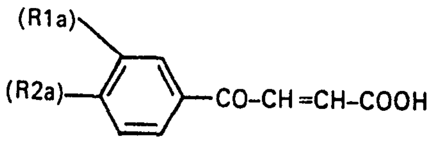 (IVa),

in which R1a and R2a have the meaning given in claim 2, with hydrazine, and in each case, if desired, subsequently converting the resulting pyridazinone Ia into the salt.

9. A process for the preparation of a compound of claim 3, which comprises

a) oxidizing an aryl-tetrahydropyridazinone of the formula IIb

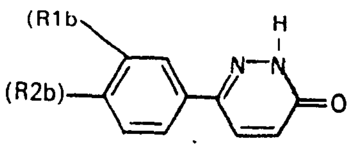 (IIb),

in which R1b and R2b have the meaning given in claim 3, or

b) reacting a morpholinobutyric acid of the formula IIIb

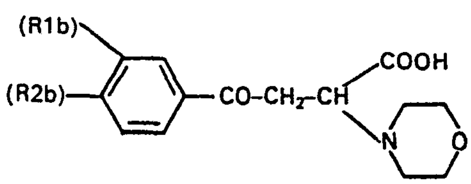 (IIIb),

in which R1b and R2b have the meaning given in claim 3, with hydrazine, or

c) reacting an acrylic acid of the formula IVb

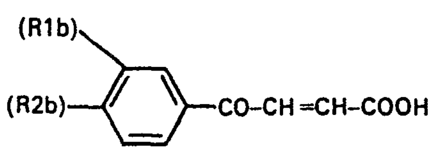 (IVb),

in which R1b and R2b have the meaning given in claim 3, with hydrazine, and in each case, if desired, subsequently converting the pyridazinone Ib thus obtained into the salt.

10. A medicament containing one or more of the compounds as claimed in claim 1.

11. The use of a compound as claimed in claim 1 in the preparation of a medicament for the treatment or prophylaxis of illnesses based on diseases of the bronchi.

12. The use of 6-(3-methoxy-4-n-propoxyphenyl)-3[2H]-pyridazinone in the preparation of a medicament for the treatment or prophylaxis of illnesses based on diseases of the bronchi.

13. The use of a compound as claimed in claim 1 in the preparation of a medicament for the treatment of illnesses based on cardiac insufficiency or for strengthening the heart.

14. The use of 6-(3-isobutoxy-4-methoxyphenyl)-3[2H]-pyridazinone in the preparation of a medicament for the treatment of illnesses based on cardiac insufficiency or for strengthening the heart.

**Claims for the contracting state AT**

1. A process for the preparation of a 6-aryl-3[2H]-pyridazinone of the general formula I

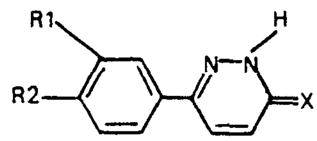 (I),

in which one of the substituents R1 or R2 denotes a methoxy group and the other denotes an alkoxy group with 2 to 5 carbon atoms or an alkenyloxy group with 3 to 5 carbon atoms and X denotes an oxygen atom or a sulfur atom, or a pharmacologically acceptable salt thereof with a base, which comprises

a) oxidizing a 6-aryl-tetrahydropyridazinone of the general formula II

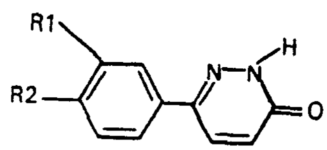 (II),

in which R1 and R2 have the abovementioned meaning, or

b) reacting a morpholinobutyric acid of the general formula III

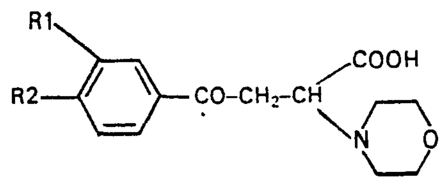 (III),

in which R1 and R2 have the abovementioned meaning, with hydrazine, or

c) reacting an acrylic acid of the general formula IV

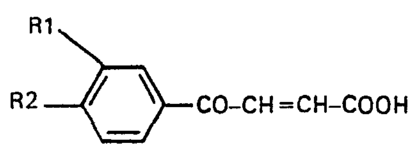 (IV),

in which R1 and R2 have the abovementioned meaning, with hydrazine, and in each case, if desired, subsequently converting the resulting pyridazinone I (X=O) into the pyridazinethione I (X–S) and/or into the salt.

2. A process for the preparation of a 6-aryl-3[2H]-pyridazinone of the general formula Ia

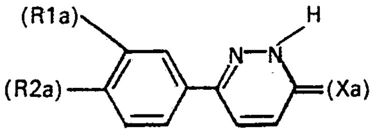

(Ia),

in which R1a denotes a methoxy group, R2a denotes an alkoxy group with 2 to 4 carbon atoms or an alkenyloxy group with 3 or 4 carbon atoms and Xa denots an oxygen atom, or a pharmacologically acceptable salt thereof with a base, which comprises

a) oxidizing an aryl-tetrahydropyridazinone of the general formula IIa

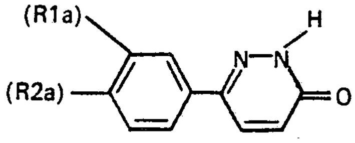

(IIa),

in which R1a and R2a have the abovementioned meaning, or

b) reacting a morpholinobutyric acid of the general formula IIIa

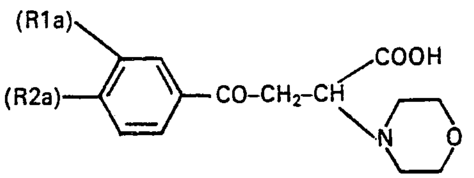

(IIIa)

in which R1a and R2a have the abovementioned meaning, with hydrazine, or

c) reacting an acrylic acid of the general formula IVa

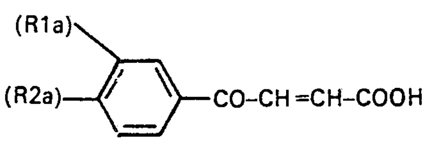

(IVa),

in which R1a and R2a have the abovementioned meaning, with hydrazine, and in each case, if desired, subsequently converting the resulting pyridazinone Ia into the salt.

3. A process for the preparation of a 6-aryl-3[2H]-pyridazinone of the general formula Ib

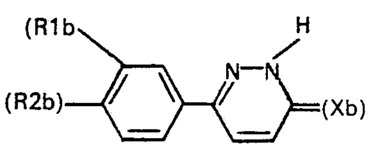

(Ib)

in which R1b denotes an alkoxy group with 2 to 4 carbon atoms or an alkenyloxy group with 3 or 4 carbon atoms, R2b denotes a methoxy group and Xb denotes an oxygen atom, or a pharmacologically acceptable salt thereof with a base, which comprises

a) oxidizing an aryl-tetrahydropyridazinone of the general formula IIb

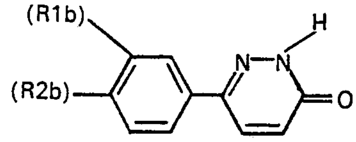

(IIb)

in which R1b and R2b have the abovementioned meaning, or

b) reacting a morpholinobutyric acid of the general formula IIIb

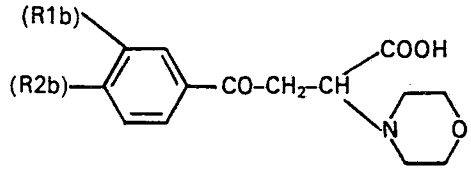

(IIIb)

in which R1b and R2b have the abovementioned meaning, with hydrazine, or

c) reacting an acrylic acid of the general formula IVb

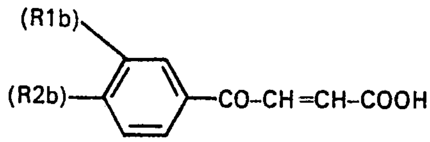

(IVb)

in which R1b and R2b have the abovementioned meaning, with hydrazine, and in each case, if desired, subsequently converting the pyridazinone Ib thus obtained into the salt.

4. A process for the preparation of 6-(3-methoxy-4-n-propoxyphenyl)-3[2H]-pyridazinone or a pharmacologically acceptable salt thereof with a base, which comprises

a) oxidizing 6-(3-methoxy-4-n-propoxyphenyl)-4,5-dihydro-3[2H]-pyridazinone, or

b) reacting 4-(3-methoxy-4-n-propoxyphenyl)-2-morpholino-4-oxobutyric acid with hydrazine, or

c) reacting 3-(3-methoxy-4-n-propoxybenzoyl)-acrylic acid with hydrazine, and, if desired, subsequently converting the resulting pyridazinone into the salt.

5. A process for the preparation of 6-(3-isobutoxy-4-methoxyphenyl)-3[2H]-pyridazinone or a pharmacologically acceptable salt thereof with a base, which comprises

a) oxidizing 6-(3-isobutoxy-4-methoxyphenyl)-4,5-dihydro-3[2H]-pyridazinone, or

b) reacting 4-(3-isobutoxy-4-methoxyphenyl)-2-morpholino-4-oxobutyric acid with hydrazine, or

c) reacting 3-(3-isobutoxy-4-methoxybenzoyl) -acrylic acid with hydrazine, and if desired, subsequently converting the resulting pyridazinone into the salt.

6. A process for the preparation of a medicament for the treatment and prophylaxis of illnesses based on diseases of the bronchi, which comprises bringing one or more 6-aryl-3[2H]pyridazinones I, Ia, Ib and/or pharmacologically acceptable salts thereof with bases into a form suitable for administration as a medicament by mixing with suitable pharmaceutically acceptable excipients.

7. The use of a 6-aryl-3[2H]-pyridazinone of the formule I given in claim 1, or a pharmacologically acceptable salt thereof with a base in the preparation of a medicament for the treatment or prophylaxis of illnesses based on diseases of the bronchi.

8. The use of 6-(3-methoxy-4-n-propoxyphenyl)-3[2H]-pyridazinone or 6-(3-isobutoxy-4-methoxyphenyl)-3[2H]-pyridazinone or a salt thereof with a base in the preparation of a medicament for the treatment of illnesses based on diseases of the bronchi.

9. The use of a 6-aryl-3[2H]-pyridazinone of the formula I given in claim 1, in which R1 and R2 have the meanings given in claim 1, or a pharmacologically acceptable salt thereof with a base in the preparation of a medicament for the treatment of illnesses based on cardiac insufficiency or for strengthening the heart.

10. The use of 6-(3-isobutoxy-4-methoxyphenyl)-3[2H] pyridazinone or a salt thereof with a base in the preparation of a medicament for the treatment of illnesses based on cardiac insufficiency or for strengthening the heart.